(19) Europäisches Patentamt
European Patent Office
Office européen des brevets

(11) EP 3 231 451 A1

(12) # EUROPEAN PATENT APPLICATION
published in accordance with Art. 153(4) EPC

(43) Date of publication:
**18.10.2017 Bulletin 2017/42**

(21) Application number: **15867040.6**

(22) Date of filing: **08.12.2015**

(51) Int Cl.:
***A61L 15/48** (2006.01)* ***A61F 13/472** (2006.01)*
***A61F 13/20** (2006.01)* ***A61F 13/53** (2006.01)*
***C08F 20/34** (2006.01)*

(86) International application number:
**PCT/JP2015/084407**

(87) International publication number:
**WO 2016/093233 (16.06.2016 Gazette 2016/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.12.2014 JP 2014249310**
**09.12.2014 JP 2014249311**
**09.12.2014 JP 2014249312**

(71) Applicant: **Kao Corporation**
**Chuo-ku**
**Tokyo 103-8210 (JP)**

(72) Inventors:
- **MATSUBARA, Shigehiro**
  **Haga-gun**
  **Tochigi 321-3497 (JP)**
- **SUZUKI, Yuka**
  **Haga-gun**
  **Tochigi 321-3497 (JP)**
- **ONOO, Makoto**
  **Haga-gun**
  **Tochigi 321-3497 (JP)**
- **TANIGUCHI, Masahiro**
  **Haga-gun**
  **Tochigi 321-3497 (JP)**

(74) Representative: **Vossius & Partner**
**Patentanwälte Rechtsanwälte mbB**
**Siebertstrasse 3**
**81675 München (DE)**

(54) **SANITARY PRODUCT AND AGENT FOR TREATING SANITARY PRODUCT**

(57) A water-soluble cationic polymer is applied to a sanitary product. The water-soluble cationic polymer has a structure including a main chain and a side chain bound to the main chain and has a molecular weight of 2000 or more. The water-soluble cationic polymer is a quaternary ammonium salt homopolymer or a quaternary ammonium salt copolymer. In the water-soluble cationic polymer, the main chain and the side chain are bound to each other at one point, and the side chain has a quaternary ammonium moiety. It is preferable that the quaternary ammonium salt homopolymer has a molecular weight of ten million or less. It is preferable that the quaternary ammonium salt copolymer is a copolymer of a polymerisable monomer having a quaternary ammonium moiety and a cationic polymerisable monomer, an anionic polymerisable monomer, or a nonionic polymerisable monomer, and has a molecular weight of ten million or less.

Fig. 1

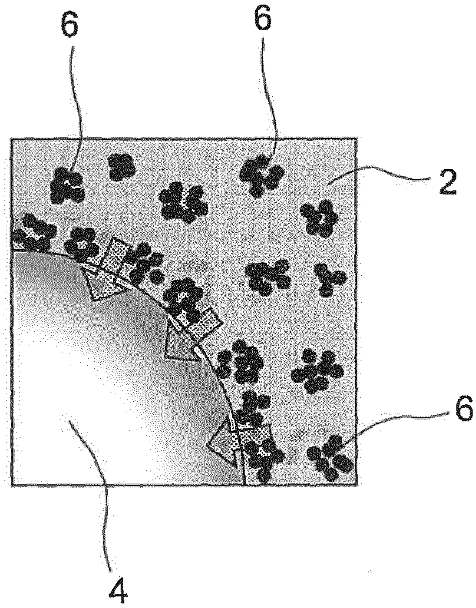


EP 3 231 451 A1

## Description

### Technical Field

The present invention relates to a sanitary product used to absorb menstrual blood. Also, the present invention relates to a treatment agent to be applied to a sanitary product used to absorb menstrual blood.

### Background Art

A technique in which a cationic macromolecular material is applied to an absorbent article to improve various properties of the absorbent article is known. For example, Patent Literature 1 describes a personal care absorbent article such as a sanitary towel or a tampon containing a porous nonwoven web material treated with a treatment agent that agglutinates or lyses erythrocytes in blood. In Patent Literature 1, a polycationic material that is a strongly positively charged polymer is used as this treatment agent. This treatment agent agglutinates or lyses erythrocytes in blood when blood enters or passes through the absorbent article.

Patent Literature 2 states that a superabsorbent polymer to be used in a sanitary towel and the like is treated with polydiallyldimethylammonium chloride, which is a cationic macromolecular material having a quaternary ammonium moiety in the side chain, thereby suppressing the evaporation of a moisture component absorbed by the superabsorbent polymer from the superabsorbent polymer.

Patent Literature 3 describes a method of manufacturing an antibacterial substance in which a cellulose material such as cotton or wood pulp and an antibacterial cationic polyelectrolyte are mixed to form non-leachable bonds therebetween. This antibacterial substance is to be used in a sanitary towel or a tampon. Polydiallyldimethylammonium chloride, for example, is used as the antibacterial cationic polyelectrolyte.

Patent Literature 4 states that a water-soluble cationic macromolecular compound is caused to be present on the surfaces of superabsorbent polymer particles to be used in a sanitary towel or the like, and thus balance between a centrifugal retention capacity and a saline flow conductivity of the superabsorbent polymer is achieved. Patent Literature 4 states that polydialkylaminoalkyl vinyl ether, polydialkylaminoalkyl (meth)acrylate, or the like is used as the water-soluble cationic macromolecular compound.

Patent Literature 5 proposes a technique in which a pyridine-based antibacterial agent, which is a cationic antibacterial agent and is not a cationic polymer, is used to impart an antibacterial property to a nonwoven fabric to be used as a constituent material of a sanitary towel. Patent Literature 5 states that when the pyridine-based antibacterial agent in which a value of inorganic value/organic value is within a specific range is used, favourable adhesion of the pyridine-based antibacterial agent to a synthetic fibre can be achieved.

### Citation List

#### Patent Literature

Patent Literature 1: JP 2002-528232A
Patent Literature 2: JP 2006-511281A
Patent Literature 3: JP 2009-506056A
Patent Literature 4: JP 2010-540207A
Patent Literature 5: JP 2003-27371A

### Summary of Invention

A sanitary product such as a sanitary towel to be used to absorb menstrual blood generally contains a superabsorbent polymer that is a hydrogel material capable of absorbing and retaining water. It is known that the water absorption speed and water absorption amount of the superabsorbent polymer vary depending on the type of water component. When a physiological saline solution and blood are compared, for example, blood has the slower absorption speed and the smaller absorption amount than a physiological saline solution. Therefore, in order to improve the properties of a sanitary product, it is important to improve various blood absorption properties of the superabsorbent polymer. However, examples of compounds that exhibit such an effect have not been shown in the related art.

The present invention provides sanitary product including a water-soluble cationic polymer that has a structure including a main chain and a side chain bound to the main chain and that has a molecular weight of 2000 or more, wherein the water-soluble cationic polymer is:

a quaternary ammonium salt homopolymer having a repeating unit represented by Formula 1 below; or
a quaternary ammonium salt copolymer having a repeating unit represented by Formula 1 below and a repeating unit represented by Formula 2 below, and

the main chain and the side chain of the water-soluble cationic polymer are bound to each other at one point, and the side chain has a quaternary ammonium moiety.

[Chem. 1]

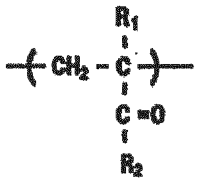
(1)

In the formula, $R_1$ represents H or $CH_3$.

$R_2$ represents 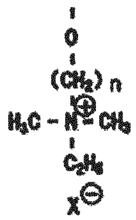 or 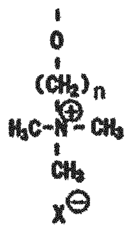 or 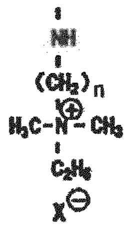 or 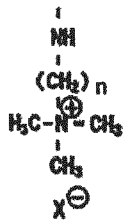 .

n represents an integer between 1 and 10.

$X^-$ represents a halide ion, $C_2H_5OSO_3^-$ or $CH_3OSO_3^-$.

[Chem. 2]

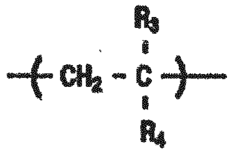 (2)

In the formula, $R_3$ represents H or $CH_3$.

$R_4$ represents 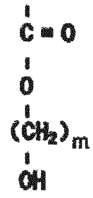 or 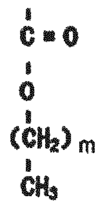 or 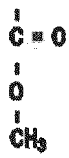 or 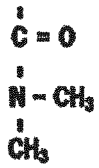 or 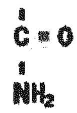 or

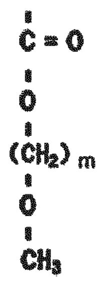 or 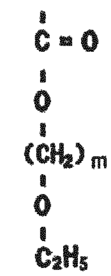 or 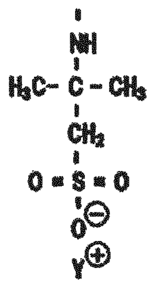 or 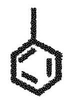.

m represents an integer between 1 and 10.

$Y^+$ represents $Na^+$ or $K^+$.

Also, the present invention provides a sanitary product to be used to absorb menstrual blood,
the sanitary product including a superabsorbent polymer,
wherein at least one member constituting the sanitary product comprises a water-soluble cationic polymer which includes a quaternary ammonium salt homopolymer or a quaternary ammonium salt copolymer,
the quaternary ammonium salt homopolymer and the quaternary ammonium salt copolymer has a streaming potential of 1500 μeq/L or more and a molecular weight of 2000 or more, and
an amount of the water-soluble cationic polymer in the sanitary product is from 0.2 $g/m^2$ to 20 $g/m^2$.

Also, the present invention provides a sanitary product that is an absorbent article including a superabsorbent polymer, a constituent member located closer to a skin of a wearer than the superabsorbent polymer, and a water-soluble cationic polymer,
the water-soluble cationic polymer being arranged closer to the skin than the constituent member, and having an agglutination speed of 0.75 mPa·s/s or less.

Furthermore, the present invention provides treatment agent for a sanitary product to be used to treat a constituent member other than a superabsorbent polymer of a sanitary product that contains the superabsorbent polymer and is to be used to absorb menstrual blood,
wherein the treatment agent contains a water-soluble cationic polymer having a molecular weight of 2000 or more,
the cationic polymer has a value of inorganic value/organic value, which expresses a ratio between an inorganic value and an organic value, of from 0.6 to 4.6, and
the cationic polymer is a quaternary ammonium salt homopolymer, a quaternary ammonium salt copolymer, or a quaternary ammonium salt polycondensate.

Brief Description of Drawings

[Fig. 1] Fig. 1 is a schematic diagram showing a menstrual blood absorbing mechanism of a sanitary product of the present invention.

[Fig. 2] Fig. 2(a) and Fig. 2(b) are schematic diagrams showing a menstrual blood absorbing mechanism of a conventional sanitary product.

Description of Embodiments

The present invention was achieved in order to improve a sanitary product, and more specifically, in order to provide a sanitary product with which various blood absorption properties of a superabsorbent polymer can be improved, and a treatment agent with which the sanitary product can be obtained.

Hereinafter, the present invention will be described based on a preferred embodiment. A sanitary product of the present invention generally includes a water-retentive absorbent member. The absorbent member contains a superabsorbent polymer that is a hydrogel material capable of absorbing and retaining water. The absorbent member may contain an absorbent fibre material in addition to the superabsorbent polymer. A liquid-permeable topsheet can be arranged on a surface of the absorbent member that faces the skin of the wearer. Moreover, a liquid-impermeable or sparingly liquid-permeable backsheet can be arranged on a non-skin-facing surface of the absorbent member. A liquid-permeable sheet called a second sheet can also be arranged between the topsheet and the absorbent member. When the sanitary product is a sanitary towel, for example, the sanitary towel generally has a longitudinally oblong shape having a longitudinal direction and a width direction orthogonal to the longitudinal direction. A pair of leak-proof cuffs extending along the longitudinal direction can be arranged on both lateral sides in the width direction on the skin-facing surface of the sanitary towel. The leak-proof cuffs tend to rise up, thereby preventing the menstrual blood excreted onto the skin-facing surface from leaking.

The absorbent member of the sanitary product includes an absorbent core containing the above-described superabsorbent polymer and a covering sheet that covers the absorbent core, for example. The covering sheet can be constituted by a liquid-permeable fibre sheet such as tissue paper or a nonwoven fabric. The same type of materials as materials conventionally used in this type of product can be used as the topsheet and the backsheet of the sanitary product without particular limitation. For example, a liquid-permeable sheet such as a nonwoven fabric or a perforated film can be used as the topsheet. A liquid-impermeable film, a spunbonded-meltblown-spunbonded laminated nonwoven fabric, which is a sparingly liquid-permeable sheet, or the like can be used as the backsheet. A plurality of pores may be formed in the liquid-impermeable film to impart a water-vapour permeating property to the film.

It is sufficient that the structure of the sanitary product is selected according to its specific application. When the sanitary product is a sanitary towel, for example, the absorbent member, topsheet, backsheet, second sheet, and the like are used as the constituent members. When the sanitary product is a tampon, an absorbent member formed in a tubular shape can be used as the constituent member. In the case where the sanitary product has any structures, the sanitary product contains the superabsorbent polymer and is treated with a treatment agent containing a cationic polymer, which will be described later.

Although a particulate superabsorbent polymer is generally used as the superabsorbent polymer to be contained in the sanitary product, a fibrous superabsorbent polymer may also be used. When a particulate superabsorbent polymer is used, its shape may be any of a spherical shape, a mass-like shape, a barrel shape, and an indefinite shape. A polymer or copolymer of acrylic acid or an alkali metal acrylate can be used as the superabsorbent polymer. Examples thereof include polyacrylic acid, polyacrylic acid salts, polymethacrylic acid, and polymethacrylic acid salts. Preferable examples of the polyacrylic acid salts and polymethacrylic acid salts include sodium polyacrylate and sodium polymethacrylate. A copolymer obtained by copolymerising a comonomer such as maleic acid, itaconic acid, acrylamide, 2-acrylamide-2-methylpropane sulphonic acid, 2-(meth)acryloylethane sulphonic acid, 2-hydroxyethyl (meth)acrylate, or styrene sulphonic acid with acrylic acid or methacrylic acid to an extent that the properties of the superabsorbent polymer are not deteriorated can also be used.

The sanitary product is treated with a treatment agent. The treatment agent is used to improve various absorption properties of the superabsorbent polymer, such as absorption speed and absorption amount. The treatment agent contains a cationic polymer. Examples of the cationic polymer include cationised cellulose and cationised starch such as starch hydroxypropyltrimonium chloride. The treatment agent can also contain a quaternary ammonium salt homopolymer, a quaternary ammonium salt copolymer, or a quaternary ammonium salt polycondensate as the cationic polymer. The term "quaternary ammonium salt" as used in the present invention encompasses a compound having a monovalent positive charge at a position of a nitrogen atom or a compound in which a monovalent positive charge is generated at a position of a nitrogen atom due to neutralisation, and specific examples thereof include salts of quaternary ammonium cations, neutralised salts of tertiary amines, and tertiary amines that become cationised in an aqueous solution. The term "quaternary ammonium moiety" described later is used in the same meaning and refers to a moiety that is positively charged in water. The term "copolymer" as used in the present invention refers to a copolymer obtained by copolymerising two or more polymerisable monomers and encompasses both binary copolymers and copolymers including three or

more components. The term "polycondensate" as used in the present invention refers to a polycondensate obtained through polymerisation of condensates including two or more monomers. When the treatment agent contains the quaternary ammonium salt homopolymer and/or the quaternary ammonium salt copolymer and/or the quaternary ammonium salt polycondensate as the cationic polymer, the treatment agent may contain at least one of the quaternary ammonium salt homopolymer, the quaternary ammonium salt copolymer, and the quaternary ammonium salt polycondensate, or a combination of any two or more of them. One type of the quaternary ammonium salt homopolymer can be used alone or two or more types thereof can be used in combination. Similarly, one type of the quaternary ammonium salt copolymer can be used alone or two or more types thereof can be used in combination. Furthermore, similarly, one type of the quaternary ammonium salt polycondensate can be used alone or two or more types thereof can be used in combination.

It is preferable to use particularly the quaternary ammonium salt homopolymer, the quaternary ammonium salt copolymer, or the quaternary ammonium salt polycondensate out of the above-described various cationic polymers from the viewpoint of the adsorption to erythrocytes. In the following description, the quaternary ammonium salt homopolymer, the quaternary ammonium salt copolymer, and the quaternary ammonium salt polycondensate are collectively referred to as "quaternary ammonium salt polymer" for the sake of convenience.

The quaternary ammonium salt homopolymer is obtained by polymeraising one type of polymerisable monomer having a quaternary ammonium moiety. On the other hand, the quaternary ammonium salt copolymer is obtained by copolymerising at least one type of polymerisable monomer having a quaternary ammonium moiety and, as necessary, at least one type of polymerisable monomer having no quaternary ammonium moiety. That is, the quaternary ammonium salt copolymer is obtained by copolymerising two or more types of polymerisable monomers having a quaternary ammonium moiety, or copolymerising one or more types of polymerisable monomers having a quaternary ammonium moiety and one or more types of polymerisable monomers having no quaternary ammonium moiety. The quaternary ammonium salt copolymer may be a random copolymer, an alternating copolymer, a block copolymer, or a graft copolymer. The quaternary ammonium salt polycondensate is obtained by polymerising a condensate including one or more types of monomers having a quaternary ammonium moiety. That is, the quaternary ammonium salt polycondensate is obtained by polymerising a condensate including two or more types of monomers having a quaternary ammonium moiety, or polycondensating a condensate including one or more types of monomers having a quaternary ammonium moiety and one or more types of monomers having no quaternary ammonium moiety.

The quaternary ammonium salt polymer is a cationic polymer having a quaternary ammonium moiety. The quaternary ammonium moiety can be formed by using an alkylating agent to make a tertiary amine into a quaternary ammonium. Alternatively, the quaternary ammonium moiety can also be formed by neutralizing a tertiary amine dissolved in acid or water. Alternatively, the quaternary ammonium moiety can be formed by using a nucleophilic reaction including a condensation reaction to form a quaternary ammonium. Examples of the alkylating agent include alkyl halides and dialkyl sulfates such as dimethyl sulfate and dimethyl sulfate. Out of these alkylating agents, dialkyl sulphates do not induce a corrosion that may occur when alkyl halides are used, and thus are preferable. Examples of the acid include hydrochloric acid, sulphuric acid, nitric acid, acetic acid, citric acid, phosphoric acid, fluorosulphonic acid, boric acid, chromic acid, lactic acid, oxalic acid, tartaric acid, gluconic acid, formic acid, ascorbic acid, and hyaluronic acid. In particular, the quaternary ammonium salt polymer obtained by making a tertiary amine moiety into a quaternary ammonium using the alkylating agent can be used to reliably neutralise the electric double layers of erythrocytes, and thus are preferable. The formation of a quaternary ammonium by a nucleophilic reaction including a condensation reaction can be performed in a manner similar to a ring-opening polycondensation reaction of dimethylamine and epichlorohydrin and a cyclisation reaction of dicyandiamide and diethylenetriamine.

The water absorption speed and water absorption amount of the superabsorbent polymer vary depending on the type of aqueous component. When a physiological saline solution and blood are compared, the blood has a slower absorption speed and a smaller absorption amount than the physiological saline solution. As a result of extensive studies on the reasons for this conducted by the inventors of the present invention, the following facts were revealed. The components of blood are broadly divided into aqueous components such as plasma and non-aqueous components such as erythrocytes, and the superabsorbent polymer absorbs the aqueous components such as plasma. As shown in Fig. 2(a), when menstrual blood 1 comes into contact with a superabsorbent polymer 4, the superabsorbent polymer 4 absorbs only an aqueous component 2 in the menstrual blood 1 and does not absorb erythrocytes, which are non-aqueous components 3. While the absorption of the aqueous component 2 into the superabsorbent polymer 4 progresses, the non-aqueous components 3 that are not absorbed into the superabsorbent polymer 4 accumulate on the surface of the superabsorbent polymer 4 to form a coating 5 as shown in Fig. 2(b). The formation of this coating 5 blocks water absorption by the superabsorbent polymer 4, and thus the absorption speed decreases. Also, the formation of this coating 5 blocks swelling of the superabsorbent polymer 4, and thus the absorption amount decreases.

As a result of extensive studies, conducted by the inventors of the present invention, on a means for preventing the occurrence of the phenomenon as shown in Fig. 2(b) to prevent the deterioration of absorption properties, it was revealed that it is effective to agglutinate erythrocytes, which make up most of the non-aqueous components in menstrual blood, to form aggregates 6 as shown in Fig. 1. The formation of the aggregates 6 of erythrocytes makes it difficult to

form a coating made of the aggregates, and even if a coating made of the aggregates is formed, spaces that the aqueous component 2 can permeate remains among the coating. Therefore, the absorption of the aqueous component 2 is unlikely to be hindered. As a result, the superabsorbent polymer 4 can sufficiently exhibit its original absorption properties. In order to further improve the absorption properties in this manner, it is preferable that the aggregates of erythrocytes have a larger particle diameter, which will be described later, and a higher hardness. Here, the absorption properties are expressed using the absorption amount and the absorption speed as criteria. The absorption amount can be expressed as a ratio between the volume of the superabsorbent polymer 4 prior to absorption and the volume of the superabsorbent polymer 4 after absorption, that is, a volume swelling rate, which will be described later. The absorption speed can be expressed as a slope of the volume swelling rate of the superabsorbent polymer 4 over time.

As a result of a study conducted by the inventors of the present invention, it was revealed that it is effective to use the above-described cationic polymer in order to form aggregates of erythrocytes in menstrual blood. The reason for this is as follows. An erythrocyte has an erythrocyte membrane on its surface. The erythrocyte membrane has a bilayer structure. This bilayer structure includes an erythrocyte membrane skeleton as a lower layer, and a lipid membrane as an upper layer. The lipid membrane exposed on the surface of the erythrocyte contains a protein called glycophorin. A sugar chain to which anionically charged sugar called sialic acid is connected is present at a terminus of the glycophorin. As a result, the erythrocyte can be treated as an anionically charged colloidal particle. An agglutinating agent is generally used to agglutinate colloidal particles. Considering that the erythrocyte is an anionic colloidal particle, it is advantageous to use a cationic substance as the agglutinating agent from the viewpoint of neutralising the electric double layer of the erythrocyte. When the agglutinating agent has a macromolecular chain, the macromolecular chains of the agglutinating agent adsorbed to the surface of the erythrocyte are likely to get entangled with one another, thus encouraging the agglutination of erythrocytes. Furthermore, the agglutinating agent having a functional group promotes the agglutination of the erythrocytes due to the interaction between the functional groups, and thus is preferable.

The action mechanism described above enables aggregates of the erythrocytes to be formed in menstrual blood. The cationic polymer has a molecular weight of preferably 2000 or more, more preferably 10000 or more, and even more preferably 30000 or more, from the viewpoint of effectively forming the aggregates of the erythrocytes. When the cationic polymer has a molecular weight that is greater than or equal to these values, the cationic polymers sufficiently become entangled with one another between the erythrocytes, or the cationic polymers are sufficiently cross-linked to one another between the erythrocytes. The upper limit value of the molecular weight is preferably ten million or less, more preferably five million or less, and even more preferably three million or less. When the cationic polymer has a molecular weight that is smaller than or equal to these values, the cationic polymer is favourably dissolved in menstrual blood. The cationic polymer has a molecular weight of preferably from 2000 to ten million, more preferably from 2000 to five million, even more preferably from 2000 to three million, further preferably from 10000 to three million, and particularly preferably from 30000 to three million. The term "molecular weight" as used in the present invention refers to a weight average molecular weight. The molecular weight of the cationic polymer can be controlled by appropriately selecting the polymerisation conditions. The molecular weight of the cationic polymer can be measured using HLC-8320GPC manufactured by Tosoh Corporation. Specific measurement conditions are as follows. As a column, a guard column $\alpha$ and an analysis column $\alpha$-M manufactured by Tosoh Corporation are connected in series and used at a column temperature of 40°C. A detector uses RI (refractive index). A measurement sample is prepared by dissolving 1 mg of the treatment agent (quaternary ammonium salt polymer) to be measured in 1 mL of an eluent. In a case of a copolymer containing a water-soluble polymerisable monomer such as hydroxyethyl methacrylate, an eluent obtained by dissolving 150 mmol/L sodium sulphate and 1 mass% of acetic acid in water is used. In the case of the copolymer containing a water-soluble polymerisable monomer such as hydroxyethyl methacrylate, a pullulan mixture obtained by dissolving 2.5 mg of pullulan having a molecular weight of 5900, 2.5 mg of pullulan having a molecular weight of 47300,2.5 mg of pullulan having a molecular weight of 212000, and 2.5 mg of pullulan having a molecular weight of 788000 in 10 mL of the eluent is used as a molecular weight standard. In the case of the copolymer containing a water-soluble polymerisable monomer such as hydroxyethyl methacrylate, the measurement is performed at a flow rate of 1.0 mL/min for an injection amount of 100 $\mu$L. In cases of copolymers other than the copolymer containing a water-soluble polymerisable monomer such as hydroxyethyl methacrylate, an eluent obtained by dissolving 50 mmol/L of lithium bromide and 1 mass% of acetic acid in a solution obtained by mixing ethanol and water at ethanol:water=3:7 (volume ratio) is used. In the cases of the copolymers other than the copolymer containing a water-soluble polymerisable monomer such as hydroxyethyl methacrylate, a mixture of polyethylene glycol (PEG) and polyethylene oxide (PEO) obtained by dissolving 10 mg of PEG having a molecular weight of 106, 10 mg of PEG having a molecular weight of 400, 10 mg of PEG having a molecular weight of 1470, 10 mg of PEG having a molecular weight of 6450, 10 mg of PEO having a molecular weight of 50000, 10 mg of PEO having a molecular weight of 235000, and 10 mg of PEO having a molecular weight of 875000 in 20 mL of the eluent is used as a molecular weight standard. In the cases of the copolymers other than the copolymer containing a water-soluble polymerisable monomer such as hydroxyethyl methacrylate, the measurement is performed at a flow rate of 0.6 mL/min and an injection amount of 100 $\mu$L.

When the quaternary ammonium salt polymer is used as the cationic polymer, the quaternary ammonium salt

polymer has a streaming potential of preferably 1500 μeq/L or more, more preferably 2000 μeq/L or more, even more preferably 3000 μeq/L or more, and even more preferably 4000 μeq/L or more, from the viewpoint of more effectively forming the aggregates of the erythrocytes. When the quaternary ammonium salt polymer has a streaming potential that is greater than or equal to these values, the electric double layer of the erythrocyte can be sufficiently neutralised. The upper limit value of the streaming potential is preferably 13000 μeq/L or less, more preferably 8000 μeq/L or less, and even more preferably 6000 μeq/L or less. When the quaternary ammonium salt polymer has a streaming potential that is smaller than or equal to these values, the electrical repulsion of the quaternary ammonium salt polymers adsorbed to the erythrocytes can be effectively prevented. The quaternary ammonium salt polymer has a streaming potential of preferably from 1500 μeq/L to 13000 μeq/L, more preferably from 2000 μeq/L to 13000 μeq/L, even more preferably from 3000 μeq/L to 8000 μeq/L, and further preferably from 4000 μeq/L to 6000 μeq/L. The streaming potential of the quaternary ammonium salt polymer can be controlled by adjusting the molecular weight of the constituent cationic monomer itself, and the copolymerisation molar ratio of a cationic monomer and an anionic monomer or a nonionic monomer that constitute the copolymer, for example. The streaming potential of the quaternary ammonium salt polymer can be measured using a streaming potential measurement apparatus (PCD04) manufactured by Spectris. Specific measurement conditions are as follows. First, in a commercially available sanitary towel, hot melt adhesive used to adhere the members to one another is inactivated using a dryer or the like, and thus the sanitary towel is disassembled into the members such as the topsheet, the absorbent member, and the backsheet. A multistage solvent extraction method using solvents ranging from a non-polar solvent to a polar solvent is performed on the disassembled members to isolate the treatment agent used in the members, and thus a solution containing a single composition is obtained. The obtained solution is dried and solidified, and the structure of the treatment agent is identified using $^1$H-NMR (nuclear magnetic resonance spectroscopy), IR (infrared spectroscopy), LC (liquid chromatography), GC (gas chromatography), MS (mass spectrometry), GPC (gel permeation chromatography), and fluorescent X-rays in combination. A measurement sample obtained by dissolving 0.001 g of the treatment agent (quaternary ammonium salt polymer) to be measured in 10 g of a physiological saline solution is titrated with a 0.001 N sodium polyethylenesulphonate solution (0.001 N polydiallyldimethylammonium chloride solution in a case where a measurement sample has a negative charge), and a titration amount X mL required until there is no electric potential difference between electrodes is measured. Thereafter, the streaming potential of the quaternary ammonium salt polymer is calculated according to Equation 1.

$$\text{Streaming potential} = (X+0.190^{*})\times 1000 \ldots \text{Equation 1}$$

(* titration amount required to titrate the physiological saline solution used as a solvent)

In order that the cationic polymer is successfully adsorbed to the surface of the erythrocyte, it is advantageous that the cationic polymer easily interacts with the above-described sialic acid. As a result of intensive studies conducted by the inventors of the present invention from this viewpoint, it was revealed that the degree of interaction between a sialic acid-binding substance and the cationic polymer can be evaluated using the value of inorganic value/organic value (referred to as "IOB (inorganic organic balance) value" hereinafter), which is a ratio of the inorganic value and organic value of a substance, as a criterion. Specifically, it was revealed that it is advantageous to use a cationic polymer having the same IOB value as that of a sialic acid-binding substance or a cationic polymer having an IOB value close to that of a sialic acid-binding substance. The sialic acid-binding substance refers to a substance having a form with which sialic acid can be present in vivo, and examples thereof include compounds in which sialic acid binds to the terminus of a glycolipid such as a galactolipid.

In general, the properties of a substance are significantly dominated by various intermolecular forces acting between molecules, and these intermolecular forces mainly include a Van Der Waals force correlated to the molecular mass and electrical affinity correlated to the molecular polarity. If the Van Der Waals force and electrical affinity, which significantly affect the change in the properties of a substance, can be found individually, the properties of unknown substances and mixtures thereof can also be predicted from a combination of the Van Der Waals force and electrical affinity. This idea is a theory that is well known as "organic conceptual diagram theory". The organic conceptual diagram theory is specifically described in "Organic Analysis" (Kaniya Shoten, 1930) by Atsushi Fujita, "Organic Qualitative Analysis: Systematic Pure Substances" (Kyoritsu Shuppan Co., Ltd., 1953) by Atsushi Fujita, "Revised Edition: Chemical Experiments - Organic Chemistry" (Kawade Shobo, 1971) by Atsushi Fujita, "Systematic Organic Qualitative Analysis (Mixture)" (Kazamashobo, 1974) by Atsushi Fujita and Masami Akatsuka, "New Edition: Organic Conceptual Diagram, Basics and Applications" (Sankyo Shuppan Co., Ltd., 2008) by Yoshio Kouda, Shiroh Satoh, and Yoshio Homma, and the like, for example. According to the organic conceptual diagram theory, regarding a physicochemical property of a substance, the degree of a property mainly arisen from a Van Der Waals force is called an "organic property", the degree of a property mainly arisen from electrical affinity is called an "inorganic property", and the properties of a substance is considered to be a combination of the "organic property" and the "inorganic property". It is defined that one carbon atom

(C) has an organic property of 20, and relative to this value, the inorganic values and organic values of various polar groups are determined as mentioned in Table 1 below. Then, the sum of the inorganic values and the sum of the organic values are determined, and the ratio between the two sums is defined as the IOB value. In the present invention, the IOB value of the above-described sialic acid-binding substance is determined based on the organic value and inorganic value, and then the IOB value of the cationic polymer is determined based on this determined value.

Table 1

| Inorganic Group | Value | Organic and Inorganic Group | Value | |
|---|---|---|---|---|
| | Inorganic | | Organic | Inorganic |
| Light Metals | 500< | $R_4$Bl-OH | 80 | 250 |
| Heavy Metals, Ammo and NH4 salt | 400< | $R_4$Sb-OH | 60 | 250 |
| $-AsO_3H_2$, $>AsSO_2H$ | 300 | $R_4$As-CH | 40 | 250 |
| $-SO_2NH$-CO-,-N=N-$NH_2$ | 260 | $R_4$P-OH | 20 | 250 |
| ⇒-N+-OH, $-SO_3H$, $-NHSO_2$-NH | 250 | -O-$So_3$H | 20 | 220 |
| -CO-NHCO-NHCO- | 250 | $>SO_2$ | 40 | 170 |
| >S-OH, -CONH-CONH-CONH-, $-SO_2NH$- | 240 | >SO | 40 | 140 |
| CS-NH-, -CONH-CO- | 230 | -CSSH | 100 | 80 |
| =N-OH-, -NHCONH- | 220 | -SCN | 90 | 80 |
| =N-NH-, -CONH-$NH_2$ | 210 | -CSOH, -COSH | 80 | 80 |
| -CONH- | 200 | -NCS | 90 | 76 |
| ->N->O | 170 | -Bl< | 80 | 70 |
| -COOH | 150 | $-NO_2$ | 70 | 70 |
| Lactone ring | 120 | -Sb< | 60 | 70 |
| -CO-O-CO- | 110 | -As<, -CN | 40 | 70 |
| Anthrathene ring, Phenanthrene ring | 105 | -P< | 20 | 70 |
| -OH | 100 | -CSS $\phi$ | 130 | 50 |
| >Hg(Organic bond) | 95 | -CSO $\phi$, -COS $\phi$ | 80 | 50 |
| -NH-NH, -O-CO-O- | 80 | -NO | 50 | 50 |
| -N<5-$NH_2$,-NH$\phi$,-N$\phi_2$)Amine | 70 | -O-$NO_2$ | 60 | 40 |
| >CO | 65 | -NC | 40 | 40 |
| -COO$\phi$, Naphthalene ring, Quinoline ring | 60 | -Sb=Sb- | 90 | 30 |
| >C=NH | 60 | -As=As- | 60 | 30 |
| -O-O- | 40 | -P=P-, -NCO | 30 | 30 |
| -N=N- | 30 | -O-NO, -SH, -S- | 40 | 20 |
| -O- | 20 | -I | 80 | 10 |
| Benzene ring(Aromatic simple ring), Pyridine ring | 15 | -Br | 60 | 10 |
| Ring(non-aromatic single ring) | 10 | -S | 50 | 10 |
| Triple bond | 3 | -Cl | 40 | 10 |
| Double bond | 2 | -F | 5 | 5 |
| -($OCH_2CH_2$)-, Sugar ring-O- | 75 | *iso* branched>- | -10 | 0 |
| | (20) | *tert* branched->- | -20 | 0 |

Specifically, when the cationic polymer is a homopolymer, the inorganic value and the organic value are determined based on a repeating unit of the homopolymer, and then the IOB value is calculated. For example, since polydiallyldimethylammonium chloride, which is a cationic polymer used in Example 1 described later, has an organic value of 160 derived from -C-×8, an inorganic value of 400 derived from Ammo and $NH_4$ salt×1, an inorganic value of 10 derived from Ring (non-aromatic single ring)×1, and an organic value of 40 and an inorganic value of 10 derived from -Cl×1=40, the sum of the inorganic values is 400+10+10=420, and the sum of the organic values is 160+40=200. Accordingly, the IOB value is 420/200=2.10.

On the other hand, when the cationic polymer is a copolymer, the IOB value is calculated according to the following procedure based on a molar ratio between monomers used in copolymerisation. That is, when the copolymer includes a monomer A and a monomer B, the monomer A has an organic value of $OR_A$ and an inorganic value of $IN_A$, the monomer B has an organic value of $OR_B$ and an inorganic value of $IN_B$, and the molar ratio monomer A/monomer B is $M_A/M_B$, the IOB value of the copolymer is calculated using the following equation.

Equation 1

$$IOB = \frac{IN_A \frac{M_A}{M_A + M_B} + IN_B \frac{M_B}{M_A + M_B}}{OR_A \frac{M_A}{M_A + M_B} + OR_B \frac{M_B}{M_A + M_B}}$$

The IOB value of the cationic polymer determined in this manner is preferably 0.6 or more, more preferably 1.8 or more, even more preferably 2.1 or more, and further preferably 2.2 or more. Also, the IOB value of the cationic polymer is preferably 4.6 or less, more preferably 3.6 or less, and even more preferably 3.0 or less. Specifically, the IOB value of the cationic polymer is preferably from 0.6 to 4.6, more preferably from 1.8 to 3.6, even more preferably from 2.1 to 3.6, and further preferably from 2.2 to 3.0. It should be noted that, regarding the IOB value of the sialic acid, the sialic acid alone has an IOB value of 4.25, and a sialic acid-binding substance has an IOB value of 3.89. The above-mentioned sialic acid-binding substance refers to a substance that sialic acid is bound to a sugar chain of glycolipid, and the sialic acid-binding substance has a higher rate of the organic value and a lower IOB value than sialic acid alone has.

When the IOB value of the cationic polymer is as described above, the organic value of the cationic polymer is preferably 40 or more, more preferably 100 or more, and even more preferably 130 or more. Also, the organic value of the cationic polymer is preferably 310 or less, more preferably 250 or less, even more preferably 240 or less, and further preferably 190 or less. For example, the organic value of the cationic polymer is preferably from 40 to 310, more preferably from 40 to 250, even more preferably from 100 to 240, and further preferably from 130 to 190. When the organic value of the cationic polymer is set to be within this range, the cationic polymer is more successfully adsorbed to the erythrocyte.

On the other hand, the inorganic value of the cationic polymer is preferably 70 or more, more preferably 90 or more, even more preferably 100 or more, further preferably 120 or more, and particularly preferably 250 or more. Also, the inorganic value is preferably 790 or less, more preferably 750 or less, even more preferably 700 or less, further preferably 680 or less, and particularly preferably 490 or less. For example, the inorganic value of the cationic polymer is preferably from 70 to 790, more preferably from 90 to 750, even more preferably from 90 to 680, further preferably from 120 to 680, and particularly preferably from 250 to 490. When the inorganic value of the cationic polymer is set to be within this range, the cationic polymer is more successfully adsorbed to the erythrocyte.

When x represents the organic value of the cationic polymer, and y represents the inorganic value thereof, it is preferable that x and y satisfy Equation A below from the viewpoint that the cationic polymer is even more successfully adsorbed to the erythrocyte.

$$y=ax \quad (A)$$

In this equation, a is preferably 0.66 or more, more preferably 0.93 or more, and even more preferably 1.96 or more. Also, a is preferably 4.56 or less, more preferably 4.19 or less, and even more preferably 3.5 or less. For example, a is preferably a numerical value of from 0.66 to 4.56, more preferably a numerical value of from 0.93 to 4.19, and even more preferably a numerical value of from 1.96 to 3.5. In particular, on a condition that the organic value and inorganic value of the cationic polymer are within the above-described ranges, when the organic value and inorganic value of the cationic polymer satisfy Equation A above, the cationic polymer easily interacts with the sialic acid-binding substance

and is thus more easily adsorbed to the erythrocyte.

It is preferable that the cationic polymer is water-soluble from the viewpoint of effectively forming the aggregates of the erythrocytes. The term "water-soluble" used in the present invention refers to a property in which, when 0.05 g of powdery cationic polymer having a diameter of 1 mm or less or a film-shaped cationic polymer having a thickness of 0.5 mm or less is added to and mixed with 50 mL of deionised water at 25°C in a 100-mL glass beaker (5 mmΦ), the whole amount of the cationic polymer is dissolved in water within 24 hours of being stirred at 600 rpm using a stirrer chip having a length of 20 mm and a width of 7 mm and a magnetic stirrer HPS-100 manufactured by As One Corporation. It should be noted that, in the present invention, more preferable solubility is solubility in which the whole amount of the cationic polymer is dissolved in the water preferably within 3 hours and more preferably within 30 minutes.

It is preferable that the cationic polymer has a structure including a main chain and a plurality of side chains bound to the main chain. In particular, it is preferable that the quaternary ammonium salt polymer has a structure including a main chain and a plurality of side chains bound to the main chain. It is preferable that the quaternary ammonium moiety is present in the side chain. In this case, when the main chain and the side chain are bound to each other at one point, the flexibility of the side chain is unlikely to be hindered, and thus the quaternary ammonium moiety present in the side chain is smoothly adsorbed to the surface of the erythrocyte. However, in the present invention, the main chain and the side chain of the cationic polymer cannot be prevented from binding to each other at two or more points. The term "binding at one point" used in the present invention means that one of the carbon atoms included in the main chain binds to one carbon atom located at the terminus of the side chain with a single bond. The term "binding at two or more points" means that two or more carbon atoms included in the main chain respectively are bound to two or more carbon atoms located at the terminus of the side chain with single bonds.

When the cationic polymer has a structure including a main chain and a plurality of side chains bound to the main chain, for example, when the quaternary ammonium salt polymer has a structure including a main chain and a plurality of side chains bound to the main chain, for example, the number of carbon atoms of each side chain is preferably four or more, more preferably five or more, and even more preferably six or more. The upper limit value of the number of carbon atoms is preferably ten or less, more preferably nine or less, and even more preferably eight or less. For example, the number of carbon atoms of the side chain is preferably four or more and ten or less, more preferably five or more and nine or less, and even more preferably six or more and eight or less. The number of carbon atoms of the side chain refers to the number of carbon atoms of the quaternary ammonium moiety (cationic moiety) in the side chain, and even when an anion, which is a counter ion, includes a carbon atom, this carbon atom is not counted. In particular, it is preferable that the number of carbon atoms between the carbon atom bound to the main chain and the carbon atom bound to the quaternary nitrogen out of the carbon atoms of the side chain is within the above-described range because steric hindrance during the adsorption of the quaternary ammonium salt polymer to the surface of the erythrocyte is reduced.

When the quaternary ammonium salt polymer is a quaternary ammonium salt homopolymer, examples of the homopolymer include polymers of a vinyl-based monomer having a quaternary ammonium moiety or a tertiary amine moiety. When the vinyl-based monomers having a tertiary amine moiety are polymerised, a quaternary ammonium salt homopolymer is obtained by making the tertiary amine moiety into a quaternary ammonium using an alkylating agent prior to and/or after the polymerisation, a tertiary amine neutralised salt is obtained by neutralising the tertiary amine moiety using acid prior to and/or after the polymerisation, or a tertiary amine cationised in an aqueous solution after the polymerisation is obtained. Examples of the alkylating agent and the acid are as described above.

In particular, it is preferable that the quaternary ammonium salt homopolymer has a repeating unit represented by Formula 1 below.

[Chem. 3]

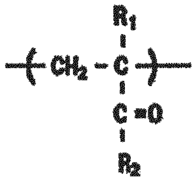 (1)

In the formula, $R_1$ represents H or $CH_3$.

$R_2$ represents 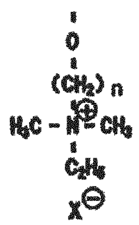 or 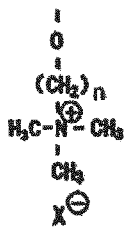 or 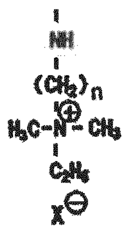 or 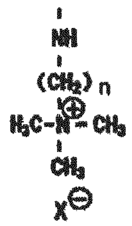 .

n represents an integer between 1 and 10.

$X^-$ represents a halide ion, $C_2H_5OSO_3^-$ or $CH_3OSO_3^-$.

A specific examples of the quaternary ammonium salt homopolymer include polyethyleneimine. Also, examples thereof include quaternary ammonium salt homopolymers in which the side chain having the quaternary ammonium moiety binds to the main chain at one point, such as poly(2-methacryloxyethyldimethylamine quaternary salt), poly(2-methacryloxyethyltrimethylammonium chloride), poly(2-methacryloxyethyldimethylethylammoniumethyl sulphate), poly(2-acryloxyethyldimethylamine quaternary salt), poly(2-acryloxyethyltrimethylamine chloride), poly(2-acryloxyethyldimethylethylammoniumethyl sulphate), poly(3-dimethylaminopropylacrylamide quaternary salt), polydimethylaminoethyl methacrylate, polyallylamine hydrochloride, cationised cellulose, polyethyleneimine, polydimethylaminopropylacrylamide, and polyamidine. On the other hand, examples of the homopolymer in which the side chain having the quaternary ammonium moiety binds to the main chain at two or more points include polydiallyldimethylammonium chloride and polydiallylamine hydrochloride.

When the quaternary ammonium salt polymer is a quaternary ammonium salt copolymer, a copolymer obtained by copolymerising two or more types of polymerisable monomers to be used in the polymerisation of the above-described quaternary ammonium salt homopolymer can be used as the copolymer. Alternatively, a copolymer obtained by copolymerising one or more types of polymerisable monomers to be used in the polymerisation of the above-described quaternary ammonium salt homopolymer and one or more types of polymerisable monomers having no quaternary ammonium moiety can be used as the quaternary ammonium salt copolymer. Furthermore, another polymerisable monomer such as $-SO_2-$ can be used in addition to or instead of the vinyl-based polymerisable monomer. The quaternary ammonium salt copolymer can be a binary copolymer or a copolymer including three or more components as described above.

In particular, it is preferable that the quaternary ammonium salt copolymer has the repeating unit represented by Formula 1 above and a repeating unit represented by Formula 2 below from the viewpoint of effectively forming the aggregates of the erythrocytes.

[Chem. 4]

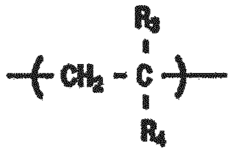 (2)

In the formula, $R_3$ represents H or $CH_3$.

$R_4$ represents 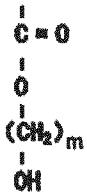 or 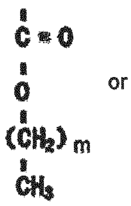 or 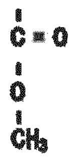 or 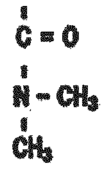 or 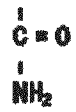 or 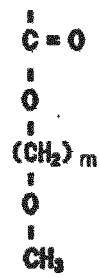 or 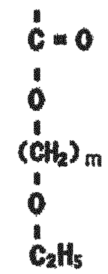 or 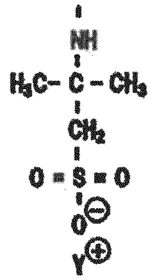 or 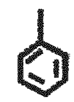.

m represents an integer between 1 and 10.

$Y^+$ represents $Na^+$ or $K^+$.

A cationic polymerizable monomer, an anionic polymerizable monomer, or a nonionic polymerizable monomer can be used as the polymerizable monomer having no quaternary ammonium moiety. In particular, the cationic polymerizable monomer or the nonionic polymerizable monomer out of these polymerizable monomers can be used to effectively agglutinate the erythrocytes because the charges are not set off against the quaternary ammonium moiety in the quaternary ammonium salt copolymer. Examples of the cationic polymerizable monomer include cyclic compounds having a nitrogen atom cationized in a specific condition, such as vinylpyridine, and linear compounds with the main chain having a nitrogen atom cationized in a specific condition, such as a condensation compound of dicyandiamide and diethylenetriamine. Examples of the anionic polymerizable monomer include 2-acrylamide-2-methylpropane sulfonic acid, methacrylic acid, acrylic acid, styrene sulfonic acid, and salts of these compounds. On the other hand, examples of the nonionic polymerizable monomer include vinyl alcohol, acrylamide, dimethylacrylamide, ethylene glycol, propylene glycol, ethylene glycol monomethacrylate, ethylene glycol monoacrylate, hydroxyethyl methacrylate, hydroxyethyl acrylate, methyl methacrylate, methyl acrylate, ethyl methacrylate, ethyl acrylate, propyl methacrylate, propyl acrylate, butyl methacrylate, and butyl acrylate. The cationic polymerizable monomer, the anionic polymerizable monomer, and the nonionic polymerizable monomer can be used alone or in combination of any two or more types of monomers. Also, two or more types of the cationic polymerizable monomers can be used in combination. Two or more types of the anionic polymerizable monomers can be used in combination. Two or more types of the nonionic polymerizable monomers can be used in combination. The molecular weight of the quaternary ammonium salt copolymer obtained by copolymerizing the cationic polymerizable monomer, the anionic polymerizable monomer and/or the nonionic polymerizable monomer as the polymerizable monomer is preferably ten million or less, more preferably five million or less, and even more preferably three million or less as described above (the same applies to quaternary ammonium salt copolymers shown as examples below).

A polymerizable monomer that has a functional group capable of forming a hydrogen bond can be used as the polymerizable monomer having no quaternary ammonium moiety. When such a polymerizable monomer is used in copolymerization, the aggregates formed by agglutinating the erythrocytes using the quaternary ammonium salt copolymer obtained by the copolymerization are likely to be hard, and therefore, the absorption property of the superabsorbent polymer is more unlikely to be hindered. Examples of the functional group capable of forming a hydrogen bond include -OH, $-NH_2$, -CHO, -COOH, -HF, and -SH. Examples of the polymerizable monomer having a functional group capable of forming a hydrogen bond include hydroxyethyl methacrylate, vinyl alcohol, acrylamide, dimethyl acrylamide, ethylene glycol, propylene glycol, ethylene glycol monomethacrylate, ethylene glycol monoacrylate, hydroxyethyl methacrylate, and hydroxyethyl acrylate. In particular, use of hydroxyethyl methacrylate, 2-hydroxyethyl methacrylate, hydroxyethyl acrylate, dimethyl acrylamide, or the like, which can form a strong hydrogen bond, makes it possible to stabilize the state in which the quaternary ammonium salt polymer is absorbed to the erythrocyte, and thus is preferable. These polymerizable monomers can be used alone or in combination of two or more.

A polymerisable monomer that has a functional group capable of making hydrophobic interaction can be used as the polymerisable monomer having no quaternary ammonium moiety. When such a polymerisable monomer is used in copolymerisation, the same advantageous effect as in the case where the above-described polymerisable monomer that has a functional group capable of forming a hydrogen bond is used, that is, the effect in which hard aggregates of the erythrocytes are likely to be formed, is exhibited. Examples of the functional group capable of making hydrophobic interaction include alkyl groups such as a methyl group, an ethyl group, and a butyl group, a phenyl group, an alkylnaphthalene group, and a fluorinated alkyl group. Examples of the polymerisable monomer having a functional group capable of making hydrophobic interaction include methyl methacrylate, methyl acrylate, ethyl methacrylate, ethyl acrylate, propyl methacrylate, propyl acrylate, butyl methacrylate, butyl acrylate, and styrene. In particular, use of methyl methacrylate, methyl acrylate, butyl methacrylate, butyl acrylate, or the like in which the effect of hydrophobic interaction is strong and that does not significantly reduce the solubility of the quaternary ammonium salt polymer, makes it possible to stabilise the state in which the quaternary ammonium salt polymer is absorbed to the erythrocyte, and thus is preferable. These polymerisable monomers can be used alone or in combination of two or more.

It is preferable that a molar ratio between the polymerisable monomer having a quaternary ammonium moiety and the polymerisable monomer having no quaternary ammonium moiety in the quaternary ammonium salt copolymer is appropriately adjusted in such a manner that the erythrocytes are sufficiently agglutinated by the quaternary ammonium salt copolymer. Alternatively, it is preferable that the molar ratio is adjusted in such a manner that the streaming potential of the quaternary ammonium salt copolymer becomes the above-described value. Alternatively, it is preferable that the molar ratio is adjusted in such a manner that the IOB of the quaternary ammonium salt copolymer becomes the above-described value. In particular, the molar ratio of the polymerisable monomer having a quaternary ammonium moiety in the quaternary ammonium salt copolymer is preferably 10 mol% or more, more preferably 22 mol% or more, even more preferably 32 mol% or more, and further preferably 38 mol% or more. Also, the molar ratio is preferably 100 mol% or less, more preferably 80 mol% or less, even more preferably 65 mol% or less, and further preferably 56 mol% or less. Specifically, the molar ratio of the polymerisable monomer having a quaternary ammonium moiety is preferably from 10 mol% to 100 mol%, more preferably from 22 mol% to 80 mol%, even more preferably from 32 mol% to 65 mol%, and further preferably from 38 mol% to 56 mol%.

When the quaternary ammonium salt polymer is a quaternary ammonium salt polycondensate, a polycondensate obtained by polymerising a condensate including one or more types of the above-described monomers having a quaternary ammonium moiety can be used as the polycondensate. Specific examples thereof include a polycondensate of dicyandiamide and diethylenetriamine and a polycondensate of dimethylamine and epichlorohydrin.

The above-described quaternary ammonium salt homopolymer and quaternary ammonium salt copolymer can be obtained by a homopolymerisation method or a copolymerisation method using a vinyl-based polymerisable monomer. Examples of the polymerisation method include radical polymerisation, living radical polymerisation, living cationic polymerisation, living anionic polymerisation, coordinated polymerisation, ring-opening polymerisation, and polycondensation. There is no particular limitation on polymerisation conditions, and it is sufficient that conditions in which a quaternary ammonium salt polymer having a desired molecular weight, streaming potential and/or IOB value can be obtained are selected appropriately.

The cationic polymer such as the quaternary ammonium salt polymer is used alone as a sanitary product, or a composition obtained by mixing the cationic polymer with other components is used as a sanitary product. Examples of components other than the cationic polymer that can be contained in this treatment agent include a solvent, a plasticiser, an aromatic agent, an antibacterial and deodorising agent, and a skin care agent. One or more of the components other than the cationic polymer that can be contained in this treatment agent can be mixed. Examples of the solvent include water, water-soluble organic solvents such as saturated aliphatic monohydric alcohol having one to four carbon atoms, and mixed solvents obtained by mixing the water-soluble organic solvent and water. Examples of the plasticiser include glycerin, polyethylene glycol, propylene glycol, ethylene glycol, and 1,3-butanediol. Examples of the aromatic agent include the aromatic agent having a green herbal aroma described in Japanese Patent No. 4776407, a plant extract, and a citrus extract. Examples of the antibacterial and deodorising agent include the cancrinite-like mineral containing a metal having an antibacterial property described in Japanese Patent No. 4526271, the porous polymer obtained by polymerising a polymerisable monomer having a phenyl group described in Japanese Patent No. 4587928, the quaternary

ammonium salt described in Japanese Patent No. 4651392, active carbon, and a clay mineral. Examples of the skin care agent include the plant extract described in Japanese Patent No. 4084278, collagen, a natural moisturising component, a moisturising agent, a keratin softening agent, and an antiphlogistic.

The rate of the cationic polymer in the above-mentioned treatment agent is preferably 1 mass% or more, more preferably 3 mass% or more, and even more preferably 5 mass% or more. Also, the rate of the cationic polymer is preferably 50 mass% or less, more preferably 30 mass% or less, and even more preferably 10 mass% or less. For example, the rate of the cationic polymer is preferably from 1 mass% to 50 mass%, more preferably from 3 mass% and to 30 mass%, and even more preferably from 5 mass% to 10 mass%. When the rate of the cationic polymer in the above-mentioned treatment agent is adjusted to be within this range, the cationic polymer in an effective amount can be applied to a sanitary product.

Constituent members of the sanitary product are treated with the above-mentioned treatment agent, and thus the cationic polymer such as the quaternary ammonium salt polymer is applied to the sanitary product. It is advantageous that the cationic polymer is applied to the sanitary product so as to come into contact with menstrual blood excreted onto the sanitary product. From this viewpoint, the cationic polymer is preferably applied to a skin-facing surface of the backsheet or to a portion of the sanitary towel located closer to a skin of a wearer than the skin-facing surface of the backsheet, more preferably applied to the absorbent member or to a portion located closer to the skin of the wearer than the absorbent member, and even more preferably applied to the covering sheet of the absorbent member or to a portion located closer to the skin of the wearer than the covering sheet. For example, it is preferable that the cationic polymer is applied to at least one of the covering sheet, the topsheet, and the second sheet arranged between the covering sheet and the topsheet of the absorbent member. It is advantageous that the cationic polymer is applied to these portions because menstrual blood can come into contact with the cationic polymer before the menstrual blood reaches the absorbent core, which is a portion including the superabsorbent polymer, thus making it possible to agglutinate the erythrocytes in the menstrual blood before the menstrual blood reaches the absorbent core. Also in the case where the absorbent core includes the cationic polymer and the case where the cationic polymer is present on the surface of the superabsorbent polymer included in the absorbent core, the effect of agglutinating the erythrocytes can be exhibited. However, the erythrocytes are more likely to be agglutinated in the case where the cationic polymer is not present near the oppositely charged anionic superabsorbent polymer than in the aforementioned cases because the elution of the cationic polymer to the menstrual blood is not suppressed, thus making it possible to obtain a sufficient agglutinating effect before the superabsorbent polymer absorbs the liquid. Therefore, an improved effect of agglutinating the erythrocytes can be obtained in the case where the cationic polymer is applied to the covering sheet, the topsheet, and/or the second sheet.

In particular, when the sanitary product including the cationic polymer has a structure including the superabsorbent polymer and constituent members located closer to a skin of wearer than the superabsorbent polymer, it is preferable that the cationic polymer is arranged in a portion of the sanitary product located closer to a skin of wearer than the constituent members. It is preferable that the cationic polymer included in the sanitary product having this structure has a blood agglutination speed of 0.75 mPa·s/s or less. The blood agglutination speed is used as a criterion of the ability of the cationic polymer to agglutinate blood to form aggregates, and the smaller the value is, the smaller the size of the aggregates after a certain period of time has passed is. That is, when the cationic polymer is used to agglutinate blood and the formed aggregates have a large size after a certain period of time, the aggregates cause clogging of the constituent members of the sanitary product and hinder the liquid-permeability, but controlling the above-mentioned agglutination speed makes it possible to increase the absorption capacity of the superabsorbent polymer without causing deterioration of the liquid-permeability due to the aggregates. Specifically, when liquid permeates the covering sheet or the second sheet in the sanitary product, the aggregate forming speed is slow, and thus clogging is unlikely to occur. Therefore, the absorption speed of the whole sanitary product is increased. Since the aggregates grow rapidly after the liquid has passed through these sheets and reached the absorbent core, the absorption capacity of the superabsorbent polymer is increased. That is, with the present invention, improvement of liquid-permeability and improvement of absorption capacity of the superabsorbent polymer, which are antinomic requirements, are fulfilled at the same time. From this viewpoint, the above-mentioned agglutination speed is more preferably 0.32 mPa·s/s or less, and even more preferably 0.15 mPa·s/s or less. The lower limit value of the agglutination speed is preferably 0.001 mPa·s/s or more, and more preferably 0.01 mPa·s/s or more.

It is preferable to use the above-described quaternary ammonium salt homopolymer or quaternary ammonium salt copolymer as a cationic polymer capable of achieving the above-mentioned agglutination speed, for example. When the quaternary ammonium salt copolymer is used, it is preferable that the quaternary ammonium salt copolymer is a copolymer of the polymerisable monomer having a quaternary ammonium moiety and the cationic polymerisable monomer, an anionic monomer, or a nonionic monomer. In particular, it is preferable that the quaternary ammonium salt copolymer is a copolymer of the polymerisable monomer having a quaternary ammonium moiety and the polymerisable monomer having a functional group capable of forming a hydrogen bond. In particular, it is preferable that the quaternary ammonium salt copolymer is a copolymer of the polymerisable monomer having a quaternary ammonium moiety and

the polymerisable monomer having no quaternary ammonium moiety, and the molar ratio of the polymerisable monomer having a quaternary ammonium moiety in the copolymer is preferably 10 mol% or more and more preferably 22 mol% or more. This molar ratio is preferably 100 mol% or less and more preferably 96 mol% or less. For example, this molar ratio is preferably from 10 mol% to 100 mol%, and more preferably from 22 mol% to 96 mol%.

The above-mentioned agglutination speed is measured using the following method. A sample obtained by adding the cationic polymer at a concentration of 500 ppm to blood (defibrinated equine blood manufactured by Nippon Bio-Test Laboratories Inc.) whose viscosity is adjusted to 8 mPa·s is used as blood to be used in the measurement of the agglutination speed. 2 μL of a physiological saline solution in which the cationic polymer has been dissolved in advance at a concentration of 5% is dripped on 200 μL of blood that has been spread on a stage in advance, and then the change in viscosity is measured using a rheometer (HAAKE RheoStress 6000 manufactured by Thermo Fisher Scientific, Inc.) with a 35 mmΦ cone plate (inclination of 1 degree) at a temperature of 30°C and a shearing speed of $10^1$ (second$^{-1}$). The change in viscosity is measured for 50 seconds, linear approximation is performed on the obtained plots, and the agglutination speed is calculated from the slope of the straight line.

The cationic polymer such as the quaternary ammonium salt polymer is applied to the constituent members of the sanitary product. The total blood permeation amount of these constituent members that is measured using a method described later is preferably 2 g or more, more preferably 3 g or more, even more preferably 4 g or more, further preferably 5 g or more, and particularly preferably 7 g or more. There is no particular limitation on the upper limit value of the total permeation amount. Use of the cationic polymer with such a total permeation amount makes it possible to control the above-described agglutination speed to be within a more preferable range.

The amount of the cationic polymer applied to the constituent members of the sanitary product is preferably 0.2 g/$m^2$ or more, more preferably 0.5 g/$m^2$ or more, even more preferably 1 g/$m^2$ or more, further preferably 3 g/$m^2$ or more, and particularly preferably 5 g/$m^2$ or more. Also, the amount the cationic polymer is preferably 20 g/$m^2$ or less, more preferably 15g/$m^2$ or less, and even more preferably 10 g/$m^2$ or less. For example, the amount of cationic polymer is preferably from 0.2 g/$m^2$ to 20 g/$m^2$, more preferably from 0.5 g/$m^2$ to 20 g/$m^2$, even more preferably from 1 g/$m^2$ to 20 g/$m^2$, further preferably from 3 g/$m^2$ to 15 g/$m^2$, and particularly preferably from 5 g/$m^2$ to 10 g/$m^2$. When the cationic polymer in an amount that is within this range is applied, the erythrocytes in excreted menstrual blood can be agglutinated effectively. It should be noted that in a case where the cationic polymer is applied to two or more portions, such as a case where the cationic polymer is applied to both the topsheet and the covering sheet, the above-mentioned amount refers to a total amount of the cationic polymer applied to those portions. When the amount of cationic polymer is 20 g/$m^2$ or less, the cationic polymer is less likely to be excessively adsorbed to the erythrocytes and is likely to suppress electrostatic repulsion, and thus agglutination is likely to occur. When the amount of cationic polymer is 0.2 g/$m^2$ or more, the cationic polymer is sufficiently adsorbed, and thus agglutination is likely to occur.

The amount of the cationic polymer applied to the constituent members of the sanitary product is preferably 0.1 mass% or more, more preferably 0.3 mass% or more, and even more preferably 0.5 mass% or more, with respect to the mass of the constituent members to which the cationic polymer is applied. Also, the amount the cationic polymer is preferably 10 mass% or less, more preferably 7.5 mass% or less, and even more preferably 5.0 mass% or less with respect to the mass of the constituent members to which the cationic polymer is to be applied. The amount of the cationic polymer is preferably from 0.1 mass% to 10 mass%, more preferably from 0.3 mass% to 7.5 mass%, and even more preferably from 0.5 mass% to 5.0 mass%, with respect to the mass of the constituent members to which the cationic polymer is applied.

The cationic polymer may be applied to the entire region of the sanitary product or a partial region of the sanitary product, in a flat view. When the cationic polymer is applied to a partial region, it is sufficient that the cationic polymer is applied to a portion facing the excretion portion of the wearer in a state in which the sanitary product is worn, for example.

When the treatment agent containing the cationic polymer is applied to the constituent members of the sanitary product, various coating methods can be used. Examples thereof include a spraying method, a dipping method, a transfer method, die coating, gravure coating, an inkjet method, and a screen printing method.

The present invention can be applied to all products to be used to absorb menstrual blood. Examples of such products include a sanitary towel, a pantyliner, and a tampon, but are not limited thereto.

Although the present invention has been described based on the preferred embodiment thereof, the present invention is not limited to the above-mentioned embodiment, and various modifications can be performed as long as the advantageous effects exhibited by the present invention are not impaired.

<1> A sanitary product comprising a water-soluble cationic polymer that has a structure including a main chain and a side chain bound to the main chain and that has a molecular weight of 2000 or more,
wherein the water-soluble cationic polymer is:

a quaternary ammonium salt homopolymer having a repeating unit represented by Formula 1 below; or
a quaternary ammonium salt copolymer having a repeating unit represented by Formula 1 below and a repeating

unit represented by Formula 2 below, and

the main chain and the side chain of the water-soluble cationic polymer are bound to each other at one point, and the side chain has a quaternary ammonium moiety.

[Chem. 5]

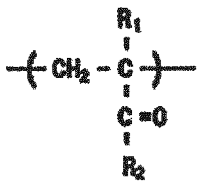

(1)

In the formula, $R_1$ represents H or $CH_3$.

$R_2$ represents 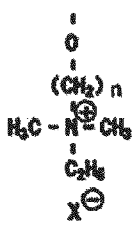 or 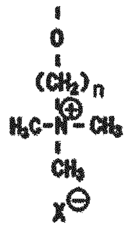 or 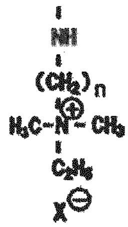 or 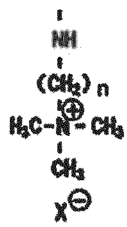 .

n represents an integer between 1 and 10.

$X^-$ represents a halide ion, $C_2H_5OSO_3^-$ or $CH_3OSO_3^-$.

[Chem. 6]

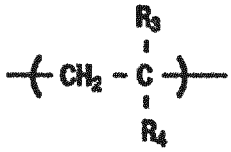 (2)

In the formula, $R_3$ represents H or $CH_3$.

$R_4$ represents 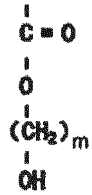 or 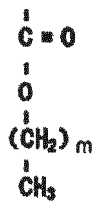 or 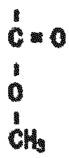 or 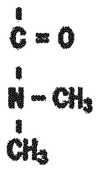 or 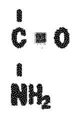 or

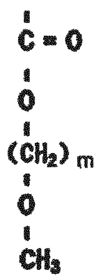 or 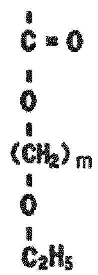 or 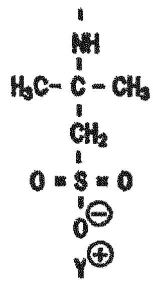 or 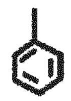 .

m represents an integer between 1 and 10.

$Y^+$ represents $Na^+$ or $K^+$.

<2> The sanitary product as set forth in <1>, wherein the side chain has four or more and ten or less carbon atoms.

<3> The sanitary product as set forth in <2>, wherein the side chain has preferably four or more, more preferably five or more, even more preferably six or more carbon atoms and preferably ten or less, more preferably nine or less, even more preferably eight or less carbon atoms.

<4> The sanitary product as set forth in any one of <1> to <3>, wherein the quaternary ammonium salt copolymer is a copolymer of a polymerisable monomer having a quaternary ammonium moiety and a cationic polymerisable monomer or a nonionic polymerisable monomer.

<5> The sanitary product as set forth in <4>, wherein the cationic polymerisable monomer is vinylpyridine or a condensation compound of dicyandiamide and diethylenetriamine.

<6> The sanitary product as set forth in <4>, wherein an anionic polymerisable monomer is 2-acrylamide-2-methylpropane sulphonic acid, methacrylic acid, acrylic acid, styrene sulphonic acid, or a salt of these compounds.

<7> The sanitary product as set forth in <4>, wherein the nonionic polymerizable monomer is vinyl alcohol, acrylamide, dimethylacrylamide, ethylene glycol, propylene glycol, ethylene glycol monomethacrylate, ethylene glycol monoacrylate, hydroxyethyl methacrylate, hydroxyethyl acrylate, methyl methacrylate, methyl acrylate, ethyl methacrylate, ethyl acrylate, propyl methacrylate, propyl acrylate, butyl methacrylate, or butyl acrylate.

<8> The sanitary product as set forth in any one of <1> to <3>, wherein the quaternary ammonium salt copolymer is a copolymer of a polymerisable monomer having a quaternary ammonium moiety and a polymerisable monomer having a functional group capable of forming a hydrogen bond.

<9> The sanitary product as set forth in <8>, wherein the functional group capable of forming a hydrogen bond is -OH, $-NH_2$, -CHO, -COOH, -HF, or -SH.

<10> The sanitary product as set forth in <8> or <9>, wherein the polymerizable monomer having a functional group capable of forming a hydrogen bond is hydroxyethyl methacrylate, vinyl alcohol, acrylamide, dimethyl acrylamide, ethylene glycol, propylene glycol, ethylene glycol monomethacrylate, ethylene glycol monoacrylate, hydroxyethyl methacrylate, or hydroxyethyl acrylate.

<11> The sanitary product as set forth in any one of <1> to <3>, wherein the quaternary ammonium salt copolymer is a copolymer of a polymerisable monomer having a quaternary ammonium moiety and a polymerisable monomer having a functional group capable of making hydrophobic interaction.
<12> The sanitary product as set forth in <11>, wherein the functional group capable of making hydrophobic interaction is an alkyl group such as a methyl group, an ethyl group, and a butyl group, a phenyl group, an alkylnaphthalene group, or a fluorinated alkyl group.
<13> The sanitary product as set forth in <11> or <12>, wherein the polymerisable monomer having a functional group capable of making hydrophobic interaction is methyl methacrylate, methyl acrylate, ethyl methacrylate, ethyl acrylate, propyl methacrylate, propyl acrylate, butyl methacrylate, butyl acrylate, or styrene.
<14> A sanitary product to be used to absorb menstrual blood,
the sanitary product comprising a superabsorbent polymer,
wherein at least one member constituting the sanitary product comprises a water-soluble cationic polymer which includes a quaternary ammonium salt homopolymer or a quaternary ammonium salt copolymer,
the quaternary ammonium salt homopolymer and the quaternary ammonium salt copolymer has a streaming potential of 1500 μeq/L or more and a molecular weight of 2000 or more, and
an amount of the water-soluble cationic polymer in the sanitary product is from 0.2 $g/m^2$ to 20 $g/m^2$.
<15> The sanitary product as set forth in <14>, wherein the quaternary ammonium salt homopolymer or the quaternary ammonium salt copolymer has a structure including a main chain and a side chain bound to the main chain, and the main chain and the side chain are bound to each other at one point.
<16> The sanitary product as set forth in <15>, wherein the side chain has four or more and ten or less carbon atoms.
<17> The sanitary product as set forth in <16>, wherein the side chain has preferably four or more, more preferably five or more, even more preferably six or more carbon atoms and preferably ten or less, more preferably nine or less, even more preferably eight or less carbon atoms.
<18> The sanitary product as set forth in any one of <14> to <17>, wherein the quaternary ammonium salt is a salt of a quaternary ammonium cation, a neutralised salt of a tertiary amine, or a tertiary amine that becomes cationised in an aqueous solution.
<19> The sanitary product as set forth in any one of <14> to <18>, wherein the quaternary ammonium salt copolymer is a copolymer obtained by copolymerising two or more polymerisable monomers and encompasses both binary copolymers and copolymers including three or more components.
<20> The sanitary product as set forth in any one of <14> to <19>, wherein the quaternary ammonium salt copolymer is obtained by copolymerising two or more types of polymerisable monomers having a quaternary ammonium moiety, or copolymerising one or more types of polymerisable monomers having a quaternary ammonium moiety and one or more types of polymerisable monomers having no quaternary ammonium moiety.
<21> The sanitary product as set forth in any one of <14> to <20>,
wherein the quaternary ammonium salt homopolymer has a repeating unit represented by Formula 1 below, and the quaternary ammonium salt copolymer has a repeating unit represented by Formula 1 below and a repeating unit represented by Formula 2 below.

[Chem. 7]

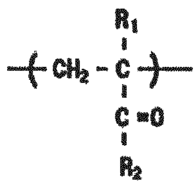 (1)

In the formula, $R_1$ represents H or $CH_3$.

$R_2$ represents 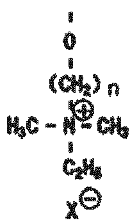 or 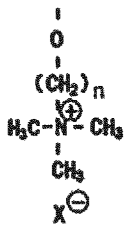 or 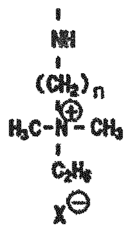 or 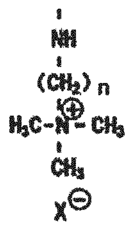 .

n represents an integer between 1 and 10.

$X^-$ represents a halide ion, $C_2H_5OSO_3^-$ or $CH_3OSO_3^-$.

[Chem. 8]

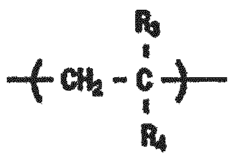 (2)

In the formula, $R_3$ represents H or $CH_3$.

$R_4$ represents 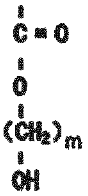 or 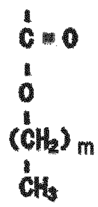 or 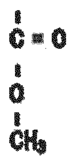 or 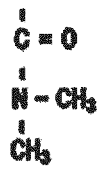 or 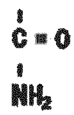 or

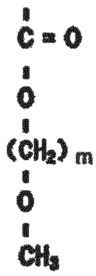 or 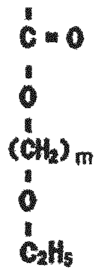 or 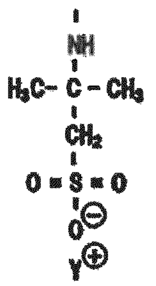 or 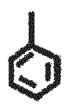 .

m represents an integer between 1 and 10.

$Y^+$ represents $Na^+$ or $K^+$.

<22> The sanitary product as set forth in any one of <15> to <21>, wherein the quaternary ammonium salt homopolymer or the quaternary ammonium salt copolymer has a streaming potential of 13000 μeq/L or less.

<23> The sanitary product as set forth in any one of <14> to <22>, wherein the quaternary ammonium salt homopolymer or the quaternary ammonium salt copolymer has a streaming potential of preferably from 1500 μeq/L to 13000 μeq/L, more preferably from 2000 μeq/L to 13000 μeq/L, even more preferably from 3000 μeq/L to 8000 μeq/L, and further preferably from 4000 μeq/L to 6000 μeq/L.

<24> The sanitary product as set forth in any one of <14> to <23>,
wherein the quaternary ammonium salt homopolymer has a molecular weight of ten million or less, or
the quaternary ammonium salt copolymer is a copolymer of a polymerisable monomer having a quaternary ammonium moiety and a cationic polymerisable monomer, an anionic polymerisable monomer, or a nonionic polymerisable monomer, and has a molecular weight of ten million or less.

<25> The sanitary product as set forth in <24>, wherein the cationic polymerisable monomer is vinylpyridine or a condensation compound of dicyandiamide and diethylenetriamine.

<26> The sanitary product as set forth in <24>, wherein the anionic polymerisable monomer is 2-acrylamide-2-methylpropane sulphonic acid, methacrylic acid, acrylic acid, styrene sulphonic acid, or a salt of these compounds.

<27> The sanitary product as set forth in <24>, wherein the nonionic polymerizable monomer is vinyl alcohol, acrylamide, dimethylacrylamide, ethylene glycol, propylene glycol, ethylene glycol monomethacrylate, ethylene glycol monoacrylate, hydroxyethyl methacrylate, hydroxyethyl acrylate, methyl methacrylate, methyl acrylate, ethyl methacrylate, ethyl acrylate, propyl methacrylate, propyl acrylate, butyl methacrylate, or butyl acrylate.

<28> A sanitary product that is an absorbent article comprising a superabsorbent polymer, a constituent member located closer to a skin of a wearer than the superabsorbent polymer, and a water-soluble cationic polymer,
the water-soluble cationic polymer being arranged in a portion of the absorbent article located closer to the skin than the constituent member, and having an agglutination speed of 0.75 mPa·s/s or less.

<29> The sanitary product as set forth in <28>, wherein the water-soluble cationic polymer has an agglutination speed of 0.32 mPa·s/s or less.

<30> The sanitary product as set forth in <28> or <29>, wherein the water-soluble cationic polymer is a quaternary ammonium salt homopolymer or a quaternary ammonium salt copolymer.

<31> The sanitary product as set forth in any one of <28> to <30>,
wherein the quaternary ammonium salt homopolymer has a molecular weight of ten million or less, or
the quaternary ammonium salt copolymer is a copolymer of a polymerisable monomer having a quaternary ammonium moiety and a cationic polymerisable monomer, an anionic polymerisable monomer, or a nonionic polymerisable monomer, and has a molecular weight of ten million or less.

<32> The sanitary product as set forth in <31>, wherein the cationic polymerisable monomer is vinylpyridine or a condensation compound of dicyandiamide and diethylenetriamine.

<33> The sanitary product as set forth in <31>, wherein the anionic polymerisable monomer is 2-acrylamide-2-methylpropane sulphonic acid, methacrylic acid, acrylic acid, styrene sulphonic acid, or a salt of these compounds.

<34> The sanitary product as set forth in <31>, wherein the nonionic polymerizable monomer is vinyl alcohol, acrylamide, dimethylacrylamide, ethylene glycol, propylene glycol, ethylene glycol monomethacrylate, ethylene glycol monoacrylate, hydroxyethyl methacrylate, hydroxyethyl acrylate, methyl methacrylate, methyl acrylate, ethyl methacrylate, ethyl acrylate, propyl methacrylate, propyl acrylate, butyl methacrylate, or butyl acrylate.

<35> The sanitary product as set forth in any one of <28> to <34>, wherein the quaternary ammonium salt copolymer is a copolymer of a polymerisable monomer having a quaternary ammonium moiety and a cationic polymerisable monomer or a nonionic polymerisable monomer.

<36> The sanitary product as set forth in any one of <28> to <35>, wherein the quaternary ammonium salt homopolymer or a quaternary ammonium salt copolymer is a quaternary ammonium formed using an alkylating agent.

<37> The sanitary product as set forth in <36>, wherein the alkylating agent is a dialkyl sulphate ester such as an alkyl halide, dimethyl sulphate and dimethyl sulphate.

<38> The sanitary product as set forth in any one of <28> to <37>, wherein the quaternary ammonium salt homopolymer or the quaternary ammonium salt copolymer is a quaternary ammonium formed using dialkyl sulphate.

<39> The sanitary product as set forth in any one of <28> to <38>, wherein the quaternary ammonium salt homopolymer or the quaternary ammonium salt copolymer has a molecular weight of 2000 or more and three million or less.

<40> The sanitary product as set forth in any one of <28> to <39>, wherein the quaternary ammonium salt copolymer is a copolymer of a polymerisable monomer having a quaternary ammonium moiety and a polymerisable monomer having a functional group capable of forming a hydrogen bond.

<41> The sanitary product as set forth in <40>, wherein the functional group capable of forming a hydrogen bond is -OH, $-NH_2$, -CHO, -COOH, -HF, or -SH.

<42> The sanitary product as set forth in <40> or <41>, wherein the polymerizable monomer having a functional group capable of forming a hydrogen bond is hydroxyethyl methacrylate, vinyl alcohol, acrylamide, dimethyl acrylamide, ethylene glycol, propylene glycol, ethylene glycol monomethacrylate, ethylene glycol monoacrylate, hydroxyethyl methacrylate, or hydroxyethyl acrylate.

<43> The sanitary product as set forth in any one of <28> to <38>, wherein the quaternary ammonium salt copolymer is a copolymer of a polymerisable monomer having a quaternary ammonium moiety and a polymerisable monomer having a functional group capable of making hydrophobic interaction.

<44> The sanitary product as set forth in <43>, wherein the functional group capable of making hydrophobic interaction is an alkyl group such as a methyl group, an ethyl group, and a butyl group, a phenyl group, an alkylnaphthalene group, or a fluorinated alkyl group.

<45> The sanitary product as set forth in <43> or <44>, wherein the polymerisable monomer having a functional group capable of making hydrophobic interaction is methyl methacrylate, methyl acrylate, ethyl methacrylate, ethyl acrylate, propyl methacrylate, propyl acrylate, butyl methacrylate, butyl acrylate, or styrene.

<46> The sanitary product as set forth in any one of <28> to <45>, wherein the quaternary ammonium salt copolymer is a copolymer of a polymerisable monomer having a quaternary ammonium moiety and a polymerisable monomer having no quaternary ammonium moiety, and a molar ratio of the polymerisable monomer having a quaternary ammonium moiety in the copolymer is 70 mol% or more.

<47> The sanitary product as set forth in any one of <28> to <46>, wherein the quaternary ammonium salt homopolymer or a quaternary ammonium salt copolymer is obtained by a homopolymerisation method or a copolymerisation method using a vinyl-based polymerisable monomer, and

the polymerisation method is radical polymerisation, living radical polymerisation, living cationic polymerisation, living anionic polymerisation, coordinated polymerisation, ring-opening polymerisation, or polycondensation.

<48> The sanitary product as set forth in any one of <1> to <47>, comprising the water-soluble cationic polymer in an amount of from 0.1 mass% to 10 mass%.

<49> The sanitary product as set forth in any one of <1> to <48>, wherein a treatment agent containing a quaternary ammonium salt homopolymer, a quaternary ammonium salt copolymer, or a quaternary ammonium salt polycondensate is applied to the sanitary product, and the treatment agent further contains a solvent, a plasticiser, an aromatic agent, an antibacterial and deodorising agent, and a skin care agent.

<50> The sanitary product as set forth in <49>, wherein water, a water-soluble organic solvent such as a saturated aliphatic monohydric alcohol having one to four carbon atoms, or a mixed solvent of the water-soluble organic solvent and water is used as the solvent.

<51> The sanitary product as set forth in <49> or <50>, wherein glycerin, polyethylene glycol, propylene glycol, ethylene glycol, or 1,3-butanediol is used as the plasticiser.

<52> The sanitary product as set forth in any one of <49> to <51>, wherein an aromatic agent having a green herbal aroma, a plant extract, or a citrus extract is used as the aromatic agent.

<53> The sanitary product as set forth in any one of <49> to <52>, wherein a cancrinite-like mineral containing a metal having an antibacterial property, a porous polymer obtained polymerising a polymerisable monomer having a phenyl group, a quaternary ammonium salt, active carbon, or a clay mineral is used as the antibacterial and deodorising agent.

<54> The sanitary product as set forth in any one of <49> to <53>, wherein a plant extract, collagen, a natural moisturising component, a moisturising agent, a keratin softening agent, or an antiphilogistic is used as the skin care agent.

<55> The sanitary product as set forth in any one of <1> to <54>, wherein the sanitary product comprises the absorbent member which includes a covering sheet, a topsheet, and a second sheet arranged between the covering sheet and the topsheet, and

a quaternary ammonium salt homopolymer, a quaternary ammonium salt copolymer, or a quaternary ammonium salt polycondensate is applied to at least one of the covering sheet, the topsheet and the second sheet.

<56> The sanitary product as set forth in any one of <1> to <55>, wherein when blood is absorbed, an aggregate of erythrocytes is formed.

<57> The sanitary product as set forth in any one of <1> to <56>, which is a sanitary towel, a pantyliner, or a tampon.

<58> A treatment agent for a sanitary product to be used to treat a constituent member other than a superabsorbent polymer of a sanitary product that contains the superabsorbent polymer and is to be used to absorb menstrual blood, wherein the treatment agent contains a water-soluble cationic polymer having a molecular weight of 2000 or more, the cationic polymer has a value of inorganic value/organic value, which expresses a ratio between an inorganic value and an organic value, of from 0.6 to 4.6, and

the cationic polymer is a quaternary ammonium salt homopolymer, a quaternary ammonium salt copolymer, or a quaternary ammonium salt polycondensate.

<59> The treatment agent for a sanitary product as set forth in <58>, wherein the cationic polymer has an IOB value of preferably from 0.6 to 4.6, more preferably from 2.1 to 3.6, and even more preferably from 2.2 to 3.0.

<60> The treatment agent for a sanitary product as set forth in <58> or <59>, wherein the quaternary ammonium

salt homopolymer or the quaternary ammonium salt copolymer is a quaternary ammonium formed using dialkyl sulphate, is formed by neutralisation by being dissolved in acid or water, or is a quaternary ammonium formed through a nucleophilic reaction including a condensation reaction.

<61> The treatment agent for a sanitary product as set forth in any one of <58> to <60>,
wherein the quaternary ammonium salt homopolymer, the quaternary ammonium salt copolymer, or the quaternary ammonium salt polycondensate is formed by neutralisation of a tertiary amine using acid, and
hydrochloric acid, sulphuric acid, nitric acid, acetic acid, citric acid, phosphoric acid, fluorosulphonic acid, boric acid, chromic acid, lactic acid, oxalic acid, tartaric acid, gluconic acid, formic acid, ascorbic acid, or hyaluronic acid is used as the acid.

<62> The treatment agent for a sanitary product as set forth in any one of <58> to <61>, wherein the value of inorganic value/organic value is from 1.8 to 3.6.

<63> The treatment agent for a sanitary product as set forth in any one of <58> to <62>, wherein the value of inorganic value/organic value is from 2.2 to 3.0.

<64> The treatment agent for a sanitary product as set forth in any one of <58> to <63>,
wherein the cationic polymer is a quaternary ammonium salt copolymer, and
the quaternary ammonium salt copolymer is a copolymer of a polymerisable monomer having a quaternary ammonium moiety and a polymerisable monomer having no quaternary ammonium moiety, and a molar ratio of the polymerisable monomer having a quaternary ammonium moiety in the copolymer is 10 mol% or more.

<65> The treatment agent for a sanitary product as set forth in any one of <58> to <64>, wherein the cationic polymer is a quaternary ammonium salt copolymer, and the quaternary ammonium salt copolymer is a copolymer of a polymerisable monomer having a quaternary ammonium moiety and a cationic polymerisable monomer or a nonionic polymerisable monomer.

<66> The treatment agent for a sanitary product as set forth in any one of <58> to <65>, wherein the cationic polymer has an organic value of from 100 to 240.

<67> The treatment agent for a sanitary product as set forth in any one of <58> to <66>, wherein the cationic polymer has an organic value of from 130 to 190.

<68> The treatment agent for a sanitary product as set forth in any one of <58> to <67>, wherein the cationic polymer has an inorganic value of from 90 to 680.

<69> The treatment agent for a sanitary product as set forth in any one of <58> to <68>, wherein the cationic polymer has an inorganic value of from 250 to 490.

<70> The treatment agent for a sanitary product as set forth in any one of <58> to <69>, wherein when x represents the organic value of the cationic polymer, and y represents the inorganic value of the cationic polymer, x and y satisfy a relationship y=ax (a is preferably 0.66 or more, more preferably 0.93 or more, and even more preferably 1.96 or more, and is preferably 4.56 or less, more preferably 4.19 or less, and even more preferably 3.5 or less).

<71> The treatment agent for a sanitary product as set forth in any one of <58 to <70, wherein the sanitary product comprises a topsheet, and the treatment agent is used to treat the topsheet.

<72> The treatment agent for a sanitary product as set forth in any one of <58> to <71>, wherein the sanitary product comprises an absorbent core and a covering sheet that covers the absorbent core, and the treatment agent is used to treat the covering sheet.

<73> The treatment agent for a sanitary product as set forth in any one of <58> to <72>, wherein the sanitary product comprises a topsheet, an absorbent member and a second sheet located between the topsheet and the absorbent member, and the treatment agent is used to treat the second sheet.

<74> A sanitary product to which the treatment agent for a sanitary product as set forth in any one of <58> to <73> is applied.

<75> The sanitary product as set forth in <74>, wherein, when blood is absorbed, an aggregate of erythrocytes is formed.

<76> The sanitary product as set forth in <74> or <75>, which is a sanitary towel, a pantyliner, or a tampon.

<77> Use of a water-soluble cationic polymer as a blood agglutinating agent,
wherein the water-soluble cationic polymer has a structure including a main chain and a side chain bound to the main chain and has a molecular weight of 2000 or more,
the water-soluble cationic polymer is:

a quaternary ammonium salt homopolymer having a repeating unit represented by Formula 1 below; and
a quaternary ammonium salt copolymer having a repeating unit represented by Formula 1 below and a repeating unit represented by Formula 2 below, and

the main chain and the side chain are bound to each other at one point, and the side chain has a quaternary ammonium moiety.

[Chem. 9]

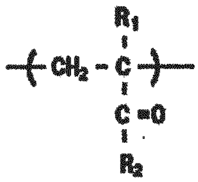

(1)

In the formula, $R_1$ represents H or $CH_3$.

$R_2$ represents 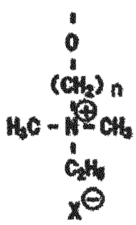 or 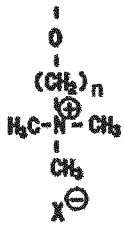 or 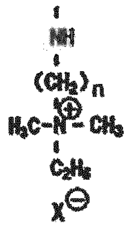 or 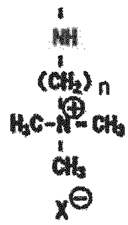 .

n represents an integer between 1 and 10.

$X^-$ represents a halide ion, $C_2H_5OSO_3^-$ or $CH_3OSO_3^-$.

[Chem. 10]

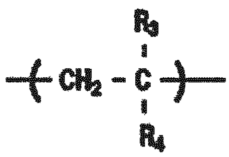

(2)

In the formula, $R_3$ represents H or $CH_3$.

$R_4$ represents 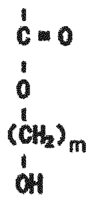 or 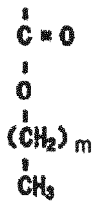 or 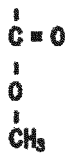 or 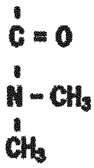 or 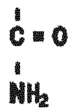 or

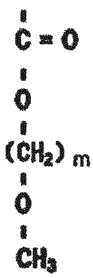 or 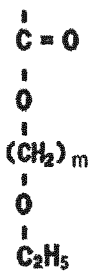 or 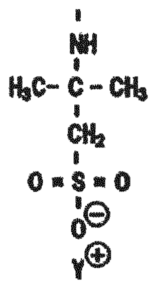 or 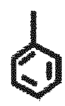 .

m represents an integer between 1 and 10.

$Y^+$ represents $Na^+$ or $K^+$.

<78> Use of a water-soluble cationic polymer as a blood agglutinating agent, the water-soluble cationic polymer being a quaternary ammonium salt homopolymer or a quaternary ammonium salt copolymer having a streaming potential of 1500 μeq/L or more and a molecular weight of 2000 or more.

<79> Use of a water-soluble cationic polymer as a blood agglutinating agent, the water-soluble cationic polymer having an agglutination speed of 0.75 mPa·s/s or less.

<80> Use of a water-soluble cationic polymer as a blood agglutinating agent,
wherein the water-soluble cationic polymer has a molecular weight of 2000 or more,
the water-soluble cationic polymer has a value of inorganic value/organic value, which expresses a ratio between an inorganic value and an organic value, of from 0.6 to 4.6, and
the water-soluble cationic polymer is a quaternary ammonium salt homopolymer, a quaternary ammonium salt copolymer, or a quaternary ammonium salt polycondensate.

Examples

Hereinafter, the present invention will be described more specifically by way of examples. However, the scope of the present invention is not limited to these examples. Unless otherwise stated, "%" and "part" means "mass%" and "part by mass", respectively.

Example 1

Polydiallyldimethylammonium chloride (Merquat 106 Polymer manufactured by The Lubrisol Corporation), which is a water-soluble quaternary ammonium salt homopolymer, was used as the quaternary ammonium salt polymer. This compound is obtained by polymerising a methyl chloride salt of diallylmethylamine as a monomer A without using another monomer (monomer B). The details of this polymer are shown in Table 2 below. An aqueous solution of a treatment agent at a concentration of 5% was prepared by dissolving this polymer in water. The agglutination speed of this polymer is shown in Table 2 below.

A 2 cm × 2 cm tissue paper having 25 $g/m^2$ was manufactured by wet papermaking using a mixture of 70 parts of High Bulk Additive manufactured by Weyerhauser and 30 parts of SKEENA PRIME manufactured by Skeena Cellulose Co.. The tissue paper had a mass of 0.068 g, a thickness of 0.326 mm under a load of 0.5 $g/cm^2$, and a bulk density of 0.08 $g/cm^3$ under a load of 0.5 $g/cm^2$. The quaternary ammonium salt polymer is adhered to the tissue paper to prepare a model sheet of the covering sheet of the absorbent member. Specifically, the quaternary ammonium salt polymer was adhered to the tissue paper by spraying the above-described 5% aqueous solution of the treatment agent at a ratio of 300% with respect to the mass of the tissue paper and then drying the tissue paper to dehydrate. The adhesion amount was 3.8 $g/m^2$. This model sheet is referred to as "Model Sheet 1". A model sheet having an adhesion amount of quaternary ammonium salt polymer of 7.5 $g/m^2$ was also manufactured by spraying the above-described 5% aqueous solution of the treatment agent at a ratio of 600% with respect to the mass of the tissue paper and then drying the tissue paper to dehydrate. This model sheet is referred to as "Model Sheet 2". In addition, a model sheet having an adhesion amount of quaternary ammonium salt polymer of 15 $g/m^2$ was also manufactured by spraying the above-described 5% aqueous solution of the treatment agent at a ratio of 1200% with respect to the mass of the tissue paper and then drying the tissue paper to dehydrate. This model sheet is referred to as "Model Sheet 3". Moreover, a model sheet having an adhesion amount of quaternary ammonium salt polymer of 1.5 $g/m^2$ was also manufactured by spraying the above-described 5% aqueous solution of the treatment agent at a ratio of 120% with respect to the mass of the tissue paper and then drying the tissue paper to dehydrate. This model sheet is referred to as "Model Sheet 4". Furthermore, a model sheet having an adhesion amount of quaternary ammonium salt polymer of 0.8 $g/m^2$ was also manufactured by spraying the above-described 5% aqueous solution of the treatment agent at a ratio of 60% with respect to the mass of the tissue paper and then drying the tissue paper to dehydrate. This model sheet is referred to as "Model Sheet 5". These model sheets were used to measure the elution amount of the quaternary ammonium salt polymer, the particle diameter of the aggregate of the erythrocytes, the hardness of the aggregate of the erythrocytes, the rate of adsorption of the quaternary ammonium salt polymer to the erythrocyte, and the volume swelling rate of a superabsorbent polymer.

The Model Sheets 1 to 5 obtained in this manner were immersed, for 30 minutes, in 3 g of blood (defibrinated equine blood manufactured by Nippon Bio-Test Laboratories Inc.) whose viscosity was adjusted to 8 mPa·s. The blood in which Model Sheet 1 was immersed contained the quaternary ammonium salt polymer at a concentration of about 500 ppm. The blood in which Model Sheet 2 was immersed contained the quaternary ammonium salt polymer at a concentration of about 1000 ppm. The blood in which Model Sheet 3 was immersed contained the quaternary ammonium salt polymer at a concentration of about 2000 ppm. The blood in which Model Sheet 4 was immersed contained the quaternary ammonium salt polymer at a concentration of about 200 ppm. The blood in which Model Sheet 5 was immersed contained the quaternary ammonium salt polymer at a concentration of about 100 ppm. Regarding the collected blood, the particle diameter of the aggregate of the erythrocytes, the hardness of the aggregate of the erythrocytes, and the

rate of adsorption of the quaternary ammonium salt polymer to the erythrocyte were measured using the following methods.

Furthermore, a superabsorbent polymer was swollen by absorbing the collected blood, and the volume swelling rate between prior to and after the absorption were measured using the following method.

Moreover, the total permeation amount and the volume swelling rate per superabsorbent polymer bead in a case of using a permeated liquid were measured using the following methods. Table 2 below shows the results.

Particle diameter of aggregate of erythrocytes

A laser diffraction/scattering particle size analyser (LA-950V2 manufactured by Horiba Ltd.) was used for the measurement. Blood was diluted to about 4000 times with a physiological saline solution, and the particle diameter of aggregate of erythrocytes was measured under the condition that the diluted solution was circulated at 0.67 L/min.

Hardness of aggregate of erythrocytes

A rheometer (MCR502 manufactured by Anton Paar GmbH) was used to for the measurement. A cone plate (CP50-1) was used as a plate. This cone plate had a diameter of 50 mm, an angle of 0.995°, and a truncation of 101 mm. The shearing speed was changed from $10^{-4}$ (1/s) to $10^{3}$ (1/s). The measurement temperature was set to 30°C, which assumes body temperature. The hardness of the aggregate of the erythrocytes was defined as a value of shearing stress at shearing speed of $10^{-2}$ (1/s).

Rate of adsorption of quaternary ammonium salt polymer to erythrocyte

Blood (defibrinated equine blood manufactured by Nippon Bio-Test Laboratories Inc.) whose viscosity was not adjusted was centrifuged at 1000×g at 4°C for 10 min, then treatment for replacing supernatant plasma with a physiological saline solution was repeated three times, and thus hemocytes that were washed with a physiological saline solution were prepared. The washed hemocytes were mixed with a physiological saline solution of the same volume to prepare a hemocyte dispersed physiological saline solution. 5 mg of the quaternary ammonium salt polymer was precisely weighed, the hemocyte dispersed physiological saline solution was added thereto such that the total mass was 10 g, and then the mixture was stirred sufficiently. After a sample was prepared, the sample was allowed to stand for 30 minutes or more and then centrifuged at 1000×g at 4°C for 10 min. 1 mL of the supernatant was collected and passed through a 5-μm filter, and then used as a sample for LC-CAD (charged aerosol detector). In this experiment, the amount of quaternary ammonium salt polymer adsorbed to the erythrocytes was calculated by quantitating the amount of non-adsorbed quaternary ammonium salt polymer and subtracting this amount from the feed amount. L-7000 manufactured by Hitachi Ltd. was used as an apparatus for LC-CAD measurement. Monoclad C18-HS 3.0 mm×50 mm manufactured by GL Sciences Inc. was used as a column, and the column temperature was set to 40°C. The measurement was performed using A: aqueous solution containing 0.1% trifluoroacetic acid, B: acetonitrile containing 0.1% trifluoroacetic acid, and C: 2-propanol as eluents in a gradient condition of A: 100% (0.0 min to 10.0 min) → B: 100% (10.1 min to 15.0 min) → C: 100% (15.1 min to 20.0 min) at a flow rate of 1.0 mL/min and an injection amount of 50 μL.

Volume swelling rate of superabsorbent polymer

A single bead of the superabsorbent polymer having a diameter of about 400 μm was placed on a glass slide, and three droplets of the collected blood were dripped on the superabsorbent polymer using a Pasteur pipette, so that the superabsorbent polymer was swollen by absorbing the blood. Cross-linked sodium polyacrylate was used as the superabsorbent polymer. After the blood was dripped, the superabsorbent polymer was sealed using a small screw cap in order to prevent moisture loss. After ten minutes elapsed, the cap was removed, and an excess amount of blood was absorbed and removed using absorbent paper. Subsequently, the diameter of the superabsorbent polymer was observed and measured under an optical microscope. When the diameter of the superabsorbent polymer prior to swelling is taken as $R_1$ (μm), and the diameter after swelling is taken as $R_2$ (μm), the volume swelling rate is defined as $(R_2/R_1)^3$.

It should be noted that the volume swelling rate is an indicator that expresses the absorption amount of the superabsorbent polymer. In addition, in this measurement, a slope of the volume swelling rate over time expresses the absorption speed of the superabsorbent polymer. In each of the examples and comparative examples, the volume swelling rate was measured ten minutes after blood was dripped, and therefore, the larger the volume swelling rate is, the faster the absorption speed is.

Total permeation amount

A 2 cm × 2 cm tissue paper having 25 $g/m^2$ was manufactured by wet papermaking using a mixture of 70 parts of High Bulk Additive manufactured by Weyerhauser and 30 parts of SKEENA PRIME manufactured by Skeena Cellulose Co.. The tissue paper had a mass of 0.068 g, a thickness of 0.326 mm under a load of 0.5 $g/cm^2$, and a bulk density of 0.08 $g/cm^3$ under a load of 0.5 $g/cm^2$. The quaternary ammonium salt polymer is adhered to the tissue paper to prepare a model sheet of the covering sheet of the absorbent member. Specifically, the above-mentioned treatment agent was sprayed at a ratio of 400% with respect to the mass of the tissue paper. The treatment agent was dried, and thus the quaternary ammonium salt polymer was adhered to the tissue paper. The adhesion amount was 5.0 $g/m^2$. This model sheet is referred to as "Model Sheet 6". Model Sheet 6 obtained in this manner was sandwiched between 35 mmΦ acrylic tubes, and 3 g of blood (defibrinated equine blood manufactured by Nippon Bio-Test Laboratories Inc.) whose viscosity was adjusted to 8 mPa·s was permeated Model Sheet 6 repeatedly three times every three minutes. The permeation amount was measured 20 seconds after each operation, and the total of the values obtained by the three operations was taken as a total permeation amount. The slower the above-described agglutination speed is, the larger the total permeation amount is, and as the total permeation amount increases, the absorption speed of a sanitary product including constituent members to which the quaternary ammonium salt polymer is adhered becomes significantly faster. It should be noted that, when an acrylic injection plate (20 cm × 10 cm; having a mass of 211 g) equipped with a 10 mmΦ tube is vertically arranged on a sanitary product, and 3 g of blood is injected into the tube repeatedly three times every three minutes, the period of time that elapses until the blood disappears in the tube is taken as the absorption speed of the sanitary product. "Every three minutes" is a time interval that takes the moment when blood is injected into the tube as a time zero.

Volume swelling rate per superabsorbent polymer bead in a case of using permeation liquid

In the experiment on the total permeation amount using the above-mentioned Model Sheet 6, all of the blood, which was permeated three times, was collected. A single bead of the superabsorbent polymer having a diameter of about 400 μm was placed on a glass slide, and three droplets of the collected blood was dripped onto the superabsorbent polymer using a Pasteur pipette, so that the superabsorbent polymer was swollen by absorbing the blood. After the blood was dripped, the superabsorbent polymer was sealed using a small screw cap in order to prevent moisture loss. After ten minutes elapsed, the cap was removed, and an excess amount of blood was absorbed and removed using absorbent paper. Subsequently, the diameter of the superabsorbent polymer was observed and measured under an optical microscope. When the diameter of the superabsorbent polymer prior to swelling is taken as $R_1$ (μm), and the diameter after swelling is taken as $R_2$ (μm), the volume swelling rate is defined as $(R_2/R_1)^3$.

Example 2

Polydiallyldimethylammonium chloride (UNISENCE FPA1000L manufactured by Senka Corporation), which is a water-soluble quaternary ammonium salt homopolymer, was used as the quaternary ammonium salt polymer. The details of this polymer are shown in Table 2. The monomer A used in this polymer is shown in Table 2. The operations were performed in the same manner as in Example 1, except that this polymer was used, and the same evaluations as those in Example 1 were performed. Table 2 shows the results.

Example 3

Polydiallyldimethylammonium chloride (Merquat 100 Polymer manufactured by The Lubrisol Corporation), which is a water-soluble quaternary ammonium salt homopolymer, was used as the quaternary ammonium salt polymer. The details of this polymer are shown in Table 2. The monomer A used in this polymer is shown in Table 2. The operations were performed in the same manner as in Example 1, except that this polymer was used, and the same evaluations as those in Example 1 were performed. Table 2 shows the results.

Example 4

Polydiallyldimethylammonium chloride (UNISENCE FPA1002L manufactured by Senka Corporation), which is a water-soluble quaternary ammonium salt homopolymer, was used as the quaternary ammonium salt polymer. The details of this polymer are shown in Table 2. The monomer A used in this polymer is shown in Table 2. The operations were performed in the same manner as in Example 1, except that this polymer was used, and the same evaluations as those in Example 1 were performed. Table 2 shows the results.

Example 5

The monomer A shown in Table 2 was dissolved in ethanol serving as a solvent, and 2,2'-Azobis(2-methylpropionamidine)dihydrochloride (V-65B manufactured by Wako Pure Chemical Industries, Ltd.), which is an oil-soluble azo initiator, was added thereto. The mixture was heated to perform polymerisation, and thus a water-soluble quaternary ammonium salt homopolymer was obtained. The details of this polymer are shown in Table 2. The monomer A used in this polymer is shown in Table 2. The operations were performed in the same manner as in Example 1, except that this polymer was used, and the same evaluations as those in Example 1 were performed. Table 2 shows the results.

Examples 6 to 9

Water-soluble quaternary ammonium salt homopolymers were obtained in the same manner as in Example 5, except that the feed amount of the monomer A to be used in Example 5 was changed. The details of these polymers are shown in Table 2. The monomer A used in these polymers is shown in Table 2. The operations were performed in the same manner as in Example 1, except that these polymers were used, and the same evaluations as those in Example 1 were performed. Table 3 shows the results.

Example 10

Poly(2-acryloxyethyltrimethylamine quaternary salt) (HP-209A manufactured by Senka Corporation), which is a water-soluble quaternary ammonium salt homopolymer, was used as the quaternary ammonium salt polymer. The details of this polymer are shown in Table 3. The monomer A used in this polymer is shown in Table 3. The operations were performed in the same manner as in Example 1, except that this polymer was used, and the same evaluations as those in Example 1 were performed. Table 3 shows the results.

Example 11

Polydimethylaminoethyl methacrylate (DIAFLOC KP201G manufactured by Mitsubishi Rayon Co., Ltd.), which is a water-soluble quaternary ammonium salt homopolymer, was used as the quaternary ammonium salt polymer. The details of this polymer are shown in Table 4. The monomer A used in this polymer is shown in Table 4. The operations were performed in the same manner as in Example 1, except that this polymer was used, and the same evaluations as those in Example 1 were performed. Table 4 shows the results.

Example 12

Polyallylamine hydrochloride (PAA-HCl-3L manufactured by Nittobo Medical Co., Ltd.), which is a water-soluble quaternary ammonium salt homopolymer, was used as the quaternary ammonium salt polymer. The details of this polymer are shown in Table 4. The monomer A used in this polymer is shown in Table 4. The operations were performed in the same manner as in Example 1, except that this polymer was used, and the same evaluations as those in Example 1 were performed. Table 4 shows the results.

Example 13

The monomer A shown in Table 4 was dissolved in ethanol serving as a solvent, and V-65B, which is an oil-soluble azo initiator, was added thereto. The mixture was heated to perform polymerisation, and thus a water-soluble quaternary ammonium salt homopolymer was obtained. The details of this polymer are shown in Table 4. The operations were performed in the same manner as in Example 1, except that this polymer was used, and the same evaluations as those in Example 1 were performed. Table 4 shows the results.

Example 14

Poly(2-methacryloxyethyldimethylethylammoniumethyl sulphate) (KAOCER MD-P manufactured by Kao Corporation), which is a water-soluble quaternary ammonium salt homopolymer, was used as the quaternary ammonium salt polymer. The details of this polymer are shown in Table 5. The monomer A used in this polymer is shown in Table 5. The operations were performed in the same manner as in Example 1, except that this polymer was used, and the same evaluations as those in Example 1 were performed. Table 5 shows the results.

Examples 15 to 18

Water-soluble quaternary ammonium salt homopolymers were obtained in the same manner as in Example 13, except that the feed amount of the monomer A to be used in Example 13 was changed. The details of these polymers are shown in Table 5. The monomer A used in these polymers is shown in Table 5. The operations were performed in the same manner as in Example 1, except that these polymers were used, and the same evaluations as those in Example 1 were performed. Table 5 shows the results.

Examples 19 and 20

The monomer A and the monomer B shown in Table 6 were used at each molar ratio shown in Table 6. These monomers were dissolved in ethanol serving as a solvent, and V-65B, which is an oil-soluble azo initiator, was added thereto. The mixture was heated to perform copolymerisation, and thus a water-soluble quaternary ammonium salt copolymer was obtained. At this time, the feed amounts of the monomer A and the monomer B were changed in respective examples. The details of these polymers are shown in Table 6. The operations were performed in the same manner as in Example 1, except that these polymers were used, and the same evaluations as those in Example 1 were performed. Table 6 shows the results.

Examples 21 to 23

The monomer A and the monomer B shown in Table 6 were used at each molar ratio shown in Table 6. These monomers were dissolved in ethanol serving as a solvent, and V-65B, which is an oil-soluble azo initiator, was added thereto. The mixture was heated to perform copolymerisation, and thus a water-soluble quaternary ammonium salt copolymer was obtained. At this time, the feed amounts of the monomer A and the monomer B were changed in respective examples. The details of these polymers are shown in Table 6. The operations were performed in the same manner as in Example 1, except that these polymers were used, and the same evaluations as those in Example 1 were performed. Table 6 shows the results.

Examples 24 to 26

The monomer A and the monomer B shown in Table 7 were used at each molar ratio shown in Table 7. These monomers were dissolved in ethanol serving as a solvent, and V-65B, which is an oil-soluble azo initiator, was added thereto. The mixture was heated to perform copolymerisation, and thus a water-soluble quaternary ammonium salt copolymer was obtained. At this time, the feed amounts of the monomer A and the monomer B were changed in respective examples. The details of these polymers are shown in Table 7. The operations were performed in the same manner as in Example 1, except that these polymers were used, and the same evaluations as those in Example 1 were performed. Table 7 shows the results.

Example 27

The monomer A and the monomer B shown in Table 7 were used at a molar ratio shown in Table 7. These monomers were dissolved in a mixed solvent of water and ethanol serving as a solvent, and V-65B, which is an oil-soluble azo initiator, was added thereto. The mixture was heated to perform copolymerisation, and thus a water-soluble quaternary ammonium salt copolymer was obtained. The details of this polymer are shown in Table 7. The operations were performed in the same manner as in Example 1, except that this polymer was used, and the same evaluations as those in Example 1 were performed. Table 7 shows the results.

Example 28

The monomer A and the monomer B shown in Table 8 were used at a molar ratio shown in Table 8. These monomers were dissolved in a mixed solvent of water and ethanol serving as a solvent, and V-65B, which is an oil-soluble azo initiator, was added thereto. The mixture was heated to perform copolymerisation, and thus a water-soluble quaternary ammonium salt copolymer was obtained. The details of this polymer are shown in Table 8. The operations were performed in the same manner as in Example 1, except that this polymer was used, and the same evaluations as those in Example 1 were performed. Table 8 shows the results.

Example 29

The monomer A and the monomer B shown in Table 8 were used at a molar ratio shown in Table 8. These monomers were dissolved in water serving as a solvent, and 2,2'-Azobis(2.4-dimethylvaleronitrile) (V-50 manufactured by Wako Pure Chemical Industries, Ltd.), which is an water-soluble azo initiator, was added thereto. The mixture was heated to perform copolymerisation, and thus a water-soluble quaternary ammonium salt copolymer was obtained. The operations were performed in the same manner as in Example 1, except that this polymer was used, and the same evaluations as those in Example 1 were performed. Table 8 shows the results.

Example 30

A copolymer of diallyldimethylammonium chloride and acrylamide (Merquat 740 Polymer manufactured by The Lubrisol Corporation), which is a water-soluble quaternary ammonium copolymer, was used. The details of this polymer are shown in Table 8. The operations were performed in the same manner as in Example 1, except that this polymer was used, and the same evaluations as those in Example 1 were performed. Table 8 shows the results.

Example 31

The monomer A and the monomer B shown in Table 9 were used at a molar ratio shown in Table 9. These monomers were dissolved in ethanol serving as a solvent, and V-65B, which is an oil-soluble azo initiator, was added thereto. The mixture was heated to perform copolymerisation, and thus a water-soluble quaternary ammonium salt copolymer was obtained. The details of this polymer are shown in Table 9 below. The operations were performed in the same manner as in Example 1, except that this polymer was used, and the same evaluations as those in Example 1 were performed. Table 9 shows the results.

Examples 32 and 33

The monomers A and the monomers B shown in Table 9 were used at each molar ratio shown in Table 9. These monomers were dissolved in ethanol serving as a solvent, and V-65B, which is an oil-soluble azo initiator, was added thereto. The mixture was heated to perform copolymerisation, and thus a water-soluble quaternary ammonium salt copolymer was obtained. The details of these polymers are shown in Table 9 below. The operations were performed in the same manner as in Example 1, except that these polymers were used, and the same evaluations as those in Example 1 were performed. Table 9 shows the results.

Example 34

Polyamidine (DIAFLOC KP7000 manufactured by Mitsubishi Rayon Co., Ltd.), which is a water-soluble quaternary ammonium salt homopolymer, was used. This compound is obtained by polymerising amidine as the monomer A without using another monomer (monomer B). The details of this polymer are shown in Table 10 below. The operations were performed in the same manner as in Example 1, except that this polymer was used, and the same evaluations as those in Example 1 were performed. Table 10 shows the results.

Example 3 5

Polyethyleneimine (EPOMIN P-1000 manufactured by Nippon Shokubai Co., Ltd.), which is a water-soluble quaternary ammonium salt homopolymer, was used. The details of this polymer are shown in Table 10 below. The monomer A used in this polymer is shown in Table 10. The operations were performed in the same manner as in Example 1, except that this polymer was used, and the same evaluations as those in Example 1 were performed. Table 10 shows the results.

Example 36

Polydiallylamine (PAS-21 manufactured by Nittobo Medical Co., Ltd.), which is a water-soluble quaternary ammonium salt homopolymer, was used. The details of this polymer are shown in Table 10 below. The monomer A used in this polymer is shown in Table 10. The operations were performed in the same manner as in Example 1, except that this polymer was used, and the same evaluations as those in Example 1 were performed. Table 10 shows the results.

Example 37

A polycondensate of dimethylamine and epichlorohydrin (UNISENCE KHE1000L manufactured by Senka Corporation), which is a water-soluble quaternary ammonium salt polycondensate, was used. The details of this polymer are shown in Table 10 below. The operations were performed in the same manner as in Example 1, except that this polymer was used, and the same evaluations as those in Example 1 were performed. Table 10 shows the results.

Example 38

Poly(diallylamine hydrochloride) (PAS-21CL manufactured by Nittobo Medical Co., Ltd.), which is a water-soluble quaternary ammonium salt homopolymer, was used. The details of this polymer are shown in Table 10 below. The monomer A used in this polymer is shown in Table 10. The operations were performed in the same manner as in Example 1, except that this polymer was used, and the same evaluations as those in Example 1 were performed. Table 10 shows the results.

Example 39

Polydiallylamine (PAA-HCl-05 manufactured by Nittobo Medical Co., Ltd.), which is a water-soluble quaternary ammonium salt homopolymer, was used. The details of this polymer are shown in Table 11 below. The monomer A used in this polymer is shown in Table 11. The operations were performed in the same manner as in Example 1, except that this polymer was used, and the same evaluations as those in Example 1 were performed. Table 11 shows the results.

Example 40

A copolymer of diallylamine hydrochloride and acrylamide (UNISENCE KCA-100L manufactured by Senka Corporation), which is a water-soluble quaternary ammonium salt copolymer, was used. The details of this polymer are shown in Table 11 below. The monomer A and the monomer B used in this polymer are shown in Table 11. The operations were performed in the same manner as in Example 1, except that this polymer was used, and the same evaluations as those in Example 1 were performed. Table 11 shows the results.

Example 41

Polyethyleneimine (EPOMIN SP-200 manufactured by Nippon Shokubai Co., Ltd.), which is a water-soluble quaternary ammonium salt homopolymer, was used. The details of this polymer are shown in Table 11 below. The monomer A used in this polymer is shown in Table 11. The operations were performed in the same manner as in Example 1, except that this polymer was used, and the same evaluations as those in Example 1 were performed. Table 11 shows the results.

Example 42

A polycondensate of dicynamimide and diethylenetriamine (UNISENCE KHP10L manufactured by Senka Corporation), which is a water-soluble quaternary ammonium salt polycondensate, was used. The details of this polymer are shown in Table 11 below. The operations were performed in the same manner as in Example 1, except that this polymer was used, and the same evaluations as those in Example 1 were performed. Table 11 shows the results.

Example 43

The monomer A and the monomer B shown in Table 11 were used at a molar ratio shown in Table 11 to perform copolymerisation, and thus a water-soluble quaternary ammonium salt copolymer was obtained. The details of this polymer are shown in Table 11 below. The operations were performed in the same manner as in Example 1, except that this polymer was used, and the same evaluations as those in Example 1 were performed. Table 11 shows the results.

Example 44

Polydiallyldimethylammoniumdiethyl sulphate (PAS-24 manufactured by Nittobo Medical Co., Ltd.), which is a water-soluble quaternary ammonium salt homopolymer, was used. The details of this polymer are shown in Table 11. The operations were performed in the same manner as in Example 1, except that this polymer was used, and the same evaluations as those in Example 1 were performed. Table 11 shows the results.

Example 45

The monomer A and the monomer B shown in Table 11 were used at a molar ratio shown in Table 11. These monomers were dissolved in ethanol serving as a solvent, and V-65B, which is an oil-soluble azo initiator, was added thereto. The mixture was heated to perform copolymerisation, and thus a water-soluble quaternary ammonium salt copolymer was obtained. The details of this polymer are shown in Table 11. The operations were performed in the same manner as in Example 1, except that this polymer was used, and the same evaluations as those in Example 1 were performed. Table 11 shows the results.

Comparative Example 1

Dimethylaminoethyl mathacrylate (manufactured by Wako Pure Chemical Industries, Ltd.) was quaternised with diethyl sulphate (manufactured by Tokyo Chemical Industry Co., Ltd.). The details of the obtained compound are shown in Table 12. The operations were performed in the same manner as in Example 1, except that this compound was used, and the same evaluations as those in Example 1 were performed. Table 12 shows the results.

Comparative Example 2

Polyethyleneimine (EPOMIN SP-018 manufactured by Nippon Shokubai Co., Ltd.), which is a water-soluble quaternary ammonium salt homopolymer, was used as the cationic polymer. The details of this polymer are shown in Table 12 below. The monomer A used in this polymer is shown in Table 12. The operations were performed in the same manner as in Example 1, except that this polymer was used, and the same evaluations as those in Example 1 were performed. Table 12 shows the results.

Comparative Example 3

In this comparative example, water-soluble cationised cellulose (MX-2075 manufactured by Kao Corporation) shown in Table 12 was used. The operations were performed in the same manner as in Example 1, except that this polymer was used, and the same evaluations as those in Example 1 were performed. Table 12 shows the results.

Table 2

| | Examples | | | | |
|---|---|---|---|---|---|
| | 1 | 2 | 3 | 4 | 5 |
| Monomer A | Methyl chloride salt of diallylmethylamine | Methyl chloride salt of diallylmethylamine | Methyl chloride salt of diallylmethylamine | Methyl chloride salt of diallylmethylamine | Methyl chloride salt of dimethylaminoethyl methacrylate |
| Monomer B | - | - | - | - | - |
| Copolymerisation molar ratio of monomer A/monomer B | 100/0 | 100/0 | 100/0 | 100/0 | 100/0 |
| Quaternary ammonium salt polycondensate | - | - | - | - | - |
| Molecular weight | 15000 | 100000 | 150000 | 600000 | 60000 |
| Organic value | 200 | 200 | 200 | 200 | 200 |
| Inorganic value | 420 | 420 | 420 | 420 | 470 |
| IOB value | 2.10 | 2.10 | 2.10 | 2.10 | 2.35 |
| Streaming potential (μeq/L) | 6700 | 7562 | 7488 | 7856 | 4995 |
| Agglutination speed (mPa·s/s) | 0.14 | - | 0.28 | 0.78 | - |
| Volume swelling rate per superabsorbent polymer bead in case of 100 ppm addition (times) | 12.1 | 17.2 | 14.2 | 13.2 | 12.2 |
| Volume swelling rate per superabsorbent polymer bead in case of 200 ppm addition (times) | 13.9 | 25.6 | 24.6 | 16.3 | 25.0 |
| Volume swelling rate per superabsorbent polymer bead in case of 500 ppm addition (times) | 25.3 | 26.1 | 28.0 | 27.4 | 25.4 |
| Volume swelling rate per superabsorbent polymer bead in case of 1000 ppm addition (times) | 26.5 | 26.4 | 25.7 | 26.5 | 26.6 |
| Volume swelling rate per superabsorbent polymer bead in case of 2000 ppm addition (times) | 24.9 | 23.6 | 22.7 | 24.1 | 26.2 |

(continued)

| | Examples | | | | |
|---|---|---|---|---|---|
| | 1 | 2 | 3 | 4 | 5 |
| Particle diameter of aggregate in case of 100 ppm addition (pm) | 7 | 9 | 8 | 7 | 8 |
| Particle diameter of aggregate in case of 200 ppm addition (μm) | 8 | 14 | 35 | 11 | 16 |
| Particle diameter of aggregate in case of 500 ppm addition (μm) | 19 | 41 | 52 | 54 | 34 |
| Particle diameter of aggretate in case of 1000 ppm addition (μm) | 23 | 29 | 48 | 39 | 36 |
| Particle diameter of aggregate in case of 2000 ppm addition (μm) | 26 | 26 | 46 | 38 | 42 |
| Hardness of aggregate in case of 500 ppm addition (mPa) | 65 | - | 175 | - | - |
| Rate of adsorption to erythrocyte in case of 500 ppm addition (%) | - | - | 40 | - | - |
| Total permeation amount (g) | 7.8 | - | 4.1 | 3.3 | 5.6 |
| Volume swelling rate per SAP bead in case of using permeation aqueous (times) | 18.7 | - | 27.2 | 25.2 | 25.3 |

Table 3

| | Examples | | | | |
|---|---|---|---|---|---|
| | 6 | 7 | 8 | 9 | 10 |
| Monomer A | Methyl chloride salt of dimethylaminoethyl methacrylate | Methyl chloride salt of dimethylaminoethyl methacrylate | Methyl chloride salt of dimethylaminoethyl methacrylate | Methyl chloride salt of dimethylaminoethyl methacrylate | Dimethyl sulphate salt of dimethylaminoethyl acrylate |
| Monomer B | - | - | - | - | - |
| Copolymerisation molar ratio of monomer A/monomer B | 100/0 | 100/0 | 100/0 | 100/0 | 100/0 |
| Quaternary ammonium salt polycondensate | - | - | - | - | - |
| Molecular weight | 95000 | 270000 | 440000 | 1130000 | 400000 |
| Organic value | 200 | 200 | 200 | 200 | 190 |
| Inorganic value | 470 | 470 | 470 | 470 | 680 |
| IOB value | 2.35 | 2.35 | 2.35 | 2.35 | 3.58 |
| Streaming potential (μeg/L) | 5842 | 5869 | 5845 | 4832 | 8544 |
| Agglutination speed (mPa·s/s) | 0.32 | - | - | - | - |
| Volume swelling rate per superabsorbent polymer bead in case of 100 ppm addition (times) | 13.5 | - | 16.6 | 14.7 | 13.0 |
| Volume swelling rate per superabsorbent polymer bead in case of 200 ppm addition (times) | 22.9 | - | 23.5 | 21.8 | 13.7 |
| Volume swelling rate per superabsorbent polymer bead in case of 500 ppm addition (times) | 26.0 | 27.2 | 24.6 | 23.5 | 23.9 |

(continued)

| | Examples | | | | |
|---|---|---|---|---|---|
| | 6 | 7 | 8 | 9 | 10 |
| Volume swelling rate per superabsorbent polymer bead in case of 1000 ppm addition (times) | 23.0 | 23.1 | 24.7 | 25.1 | 22.1 |
| Volume swelling rate per superabsorbent polymer bead in case of 2000 ppm addition (times) | 23.5 | 26.6 | 23.4 | 24.5 | 27.5 |
| Particle diameter of aggregate in case of 100 ppm addition (μm) | 9 | - | 10 | 8 | 7 |
| Particle diameter of aggregate in case of 200 ppm addition (μm) | 18 | - | 25 | 21 | 7 |
| Particle diameter of aggregate in case of 500 ppm addition (μm) | 36 | 52 | 48 | 41 | 78 |
| Particle diameter of aggregate in case of 1000 ppm addition (μm) | 40 | 42 | 47 | 42 | 73 |
| Particle diameter of aggregate in case of 2000 ppm addition (μm) | 44 | 42 | 38 | 45 | 57 |
| Hardness of aggregate in case of 500 ppm addition (mPa) | 102 | - | - | - | - |
| Rate of adsorption to erythrocyte in case of 500 ppm addition (%) | | | - | - | - |
| Total permeation amount (g) | 5.5 | - | - | - | 3.6 |

(continued)

| | Examples | | | | |
|---|---|---|---|---|---|
| | 6 | 7 | 8 | 9 | 10 |
| Volume swelling rate per SAP bead in case of using permeation aqueous (times) | 26.2 | - | - | - | 26.9 |

Table 4

| | Examples | | |
|---|---|---|---|
| | 11 | 12 | 13 |
| Monomer A | Dimethylaminoethyl methacrylate | Allylamine hydrochloride | Dimethyl sulphate salt of dimethylaminoethyl methacrylate |
| Monomer B | - | - | - |
| Copolymerisation molar ratio of monomer A/monomer B | 100/0 | 100/0 | 100/0 |
| Quaternary ammonium salt polycondensate | - | - | - |
| Molecular weight | 3000000 | 15000 | 19000 |
| Organic value | 140 | 90 | 240 |
| Inorganic value | 130 | 410 | 680 |
| IOB value | 0.93 | 4.56 | 2.83 |
| Streaming potential (μeq/L) | 5396 | 12266 | 3980 |
| Agglutination speed (mPa·s/s) | - | - | - |
| Volume swelling rate per superabsorbent polymer bead in case of 100 ppm addition (times) | - | 13.9 | 12.5 |
| Volume swelling rate per superabsorbent polymer bead in case of 200 ppm addition (times) | - | 13.8 | - |
| Volume swelling rate per superabsorbent polymer bead in case of 500 ppm addition (times) | 23.9 | 15.7 | 13.2 |
| Volume swelling rate per superabsorbent polymer bead in case of 1000 ppm addition (times) | 23.7 | 21.7 | 21.5 |
| Volume swelling rate per superabsorbent polymer bead in case of 2000 ppm addition (times) | 22.3 | 22.8 | 25.7 |
| Particle diameter of aggregate in case of 100 ppm addition (μm) | - | 7 | 7 |
| Particle diameter of aggregate in case of 200 ppm addition (μm) | - | 8 | - |
| Particle diameter of aggregate in case of 500 ppm addition (μm) | 78 | 16 | 8 |
| Particle diameter of aggregate in case of 1000 ppm addition (μm) | 55 | 22 | 10 |
| Particle diameter of aggregate in case of 2000 ppm addition (μm) | 16 | 54 | 26 |
| Hardness of aggregate in case of 500 ppm addition (mPa) | - | - | 8 |
| Rate of adsorption to erythrocyte in case of 500 ppm addition (%) | - | - | - |

(continued)

| | Examples | | |
|---|---|---|---|
| | 11 | 12 | 13 |
| Total permeation amount (g) | - | - | - |
| Volume swelling rate per SAP bead in case of using permeation aqueous (times) | - | - | - |

Table 5

| | Examples | | | | |
|---|---|---|---|---|---|
| | 14 | 15 | 16 | 17 | 18 |
| Monomer A | Diethyl sulphate salt of dimethylaminoethyl methacrylate | Diethyl sulphate salt of dimethylaminoethyl methacrylate | Diethyl sulphate salt of dimethylaminoethyl methacrylate | Diethyl sulphate salt of dimethylaminoethyl methacrylate | Diethyl sulphate salt of dimethylaminoethyl methacrylate |
| Monomer B | - | - | - | - | - |
| Copolymerisation molar ratio of monomer A/monomer B | 100/0 | 100/0 | 100/0 | 100/0 | 100/0 |
| Quaternary ammonium salt polycondensate | - | - | - | - | - |
| Molecular weight | 56000 | 34000 | 170000 | 2800000 | 420000 |
| Organic value | 240 | 240 | 240 | 240 | 240 |
| Inorganic value | 680 | 680 | 680 | 680 | 680 |
| IOB value | 2.83 | 2.83 | 2.83 | 2.83 | 2.83 |
| Streaming potential (μeq/L) | 4561 | 4054 | 4034 | 4449 | 4468 |
| Agglutination speed (mPa·s/s) | 0.20 | - | 0.75 | - | 0.98 |
| Volume swelling rate per superabsorbent polymer bead in case of 100 ppm addition (times) | 10.8 | 15.3 | 10.8 | 25.1 | 25.8 |
| Volume swelling rate per superabsorbent polymer bead in case of 200 ppm addition (times) | 10.5 | - | 12.8 | - | - |
| Volume swelling rate per superabsorbent polymer bead in case of 500 ppm addition (times) | 24.5 | 22.0 | 25.5 | 24.9 | 26.4 |

(continued)

| | Examples | | | | |
|---|---|---|---|---|---|
| | 14 | 15 | 16 | 17 | 18 |
| Volume swelling rate per superabsorbent polymer bead in case of 1000 ppm addition (times) | 23.8 | 22.4 | 26.7 | 26.7 | 27.4 |
| Volume swelling rate per superabsorbent polymer bead in case of 2000 ppm addition (times) | 25.0 | 26.0 | 27.4 | 27.1 | 26.2 |
| Particle diameter of aggregate in case of 100 ppm addition (μm) | 7 | 9 | 7 | 44 | 29 |
| Particle diameter of aggregate in case of 200 ppm addition (μm) | 10 | - | 10 | - | - |
| Particle diameter of aggregate in case of 500 ppm addition (μm) | 31 | 13 | 36 | 46 | 55 |
| Particle diameter of aggregate in case of 1000 ppm addition (μm) | 28 | 18 | 32 | 62 | 70 |
| Particle diameter of aggregate in case of 2000 ppm addition (μm) | 31 | 32 | 32 | 39 | 38 |
| Hardness of aggregate in case of 500 ppm addition (mPa) | 73 | 14 | 124 | 152 | 115 |
| Rate of adsorption to erythrocyte in case of 500 ppm addition (%) | - | - | 74 | - | - |

(continued)

| | Examples | | | | |
|---|---|---|---|---|---|
| | 14 | 15 | 16 | 17 | 18 |
| Total permeation amount (g) | 5.6 | - | 4.5 | - | 3.8 |
| Volume swelling rate per SAP bead in case of using permeation aqueous (times) | 15.1 | - | 24.6 | - | 18 |

Table 6

| | Examples | | | | |
|---|---|---|---|---|---|
| | 19 | 20 | 21 | 22 | 23 |
| Monomer A | Diethyl sulphate salt of dimethylaminoethyl methacrylate | Diethyl sulphate salt of dimethylaminoethyl methacrylate | Diethyl sulphate salt of dimethylaminoethyl methacrylate | Diethyl sulphate salt of dimethylaminoethyl methacrylate | Dimethyl sulphate salt of dimethylaminoethyl methacrylate |
| Monomer B | Dimetylacrylamide | Dimetylacrylamide | 2-Hydroxyethyl methacrylate | 2-hydroxyethyl methacrylate | 2-hydroxyethyl methacrylate |
| Copolymerisation molar ratio of monomer A/monomer B | 32/68 | 56/44 | 63/37 | 38/62 | 22/78 |
| Quaternary ammonium salt polycondensate | - | - | - | - | - |
| Molecular weight | 170000 | 200000 | 370000 | 260000 | 340000 |
| Organic value | 138 | 174 | 188.2 | 153.2 | 130.8 |
| Inorganic value | 309.4 | 440.2 | 487.6 | 357.6 | 274.4 |
| IOB value | 2.24 | 2.53 | 2.59 | 2.33 | 2.10 |
| Streaming potential (μeg/L) | 6755 | 5174 | 4374 | 3446 | 3658 |
| Agglutination speed (mPa·s/s) | 0.17 | 0.48 | 0.82 | 0.16 | 0.03 |
| Volume swelling rate per superabsorbent polymer bead in case of 100 ppm addition (times) | 16.9 | 12.4 | 24.0 | 11.6 | 19.5 |
| Volume swelling rate per superabsorbent polymer bead in case of 200 ppm addition (times) | 24.7 | 20.5 | - | 20.3 | 19.8 |

(continued)

| | Examples | | | | |
|---|---|---|---|---|---|
| | 19 | 20 | 21 | 22 | 23 |
| Volume swelling rate per superabsorbent polymer bead in case of 500 ppm addition (times) | 26.4 | 24.9 | 24.4 | 24.9 | 23.7 |
| Volume swelling rate per superabsorbent polymer bead in case of 1000 ppm addition (times) | 24.7 | 23.1 | 22.8 | 26.7 | 21.6 |
| Volume swelling rate per superabsorbent polymer bead in case of 2000 ppm addition (times) | 22.9 | 25.3 | 23.4 | 22.2 | 20.4 |
| Particle diameter of aggregate in case of 100 ppm addition (μm) | 6 | 7 | 41 | 6 | 8 |
| Particle diameter of aggregate in case of 200 ppm addition (μm) | 10 | 10 | - | 11 | 8 |
| Particle diameter of aggregate in case of 500 ppm addition (μm) | 32 | 37 | 69 | 31 | 18 |
| Particle diameter of aggregate in case of 1000 ppm addition (μm) | 30 | 41 | 63 | 37 | 17 |
| Particle diameter of aggregate in case of 2000 ppm addition (μm) | 42 | 45 | 40 | 33 | 26 |
| Hardness of aggregate in case of 500 ppm addition (mPa) | - | - | - | 163 | - |

(continued)

| | Examples | | | | |
|---|---|---|---|---|---|
| | 19 | 20 | 21 | 22 | 23 |
| Rate of adsorption to erythrocyte in case of 500 ppm addition (%) | - | - | - | - | - |
| Total permeation amount (g) | 5.5 | 3.9 | 3.8 | 2.3 | 7.1 |
| Volume swelling rate per SAP bead in case of using permeation aqueous (times) | 25.4 | 27.6 | 27.2 | 22.5 | 22.1 |

Table 7

| | Examples | | | |
|---|---|---|---|---|
| | 24 | 25 | 26 | 27 |
| Monomer A | Diethyl sulphate salt of dimethylaminoethyl methacrylate | Diethyl sulphate salt of dimethylaminoethyl methacrylate | Diethyl sulphate salt of dimethylaminoethyl methacrylate | Diethyl sulphate salt of dimethylaminoethyl methacrylate |
| Monomer B | Metyl methacrylate | Metyl methacrylate | Metyl methacrylate | Sodium 2-acrylamide-2-methylpropane sulphonate |
| Copolymerisation molar ratio of monomer A/monomer B | 56/44 | 56/44 | 56/44 | 60/40 |
| Quaternary ammonium salt polycondensate | - | - | - | - |
| Molecular weight | 83000 | 215000 | 287000 | 330000 |
| Organic value | 169.6 | 169.6 | 169.6 | 1.88 |
| Inorganic value | 407 | 407 | 407 | 788 |
| IOB value | 2.40 | 2.40 | 2.40 | 4.19 |
| Streaming potential (μeg/L) | 4286 | 4590 | 4002 | 2268 |
| Agglutination speed (mPa·s/s) | 0.26 | 1.39 | - | - |
| Volume swelling rate per superabsorbent polymer bead in case of 100 ppm addition (times) | 14.5 | 11.4 | - | 20.0 |
| Volume swelling rate per superabsorbent polymer bead in case of 200 ppm addition (times) | 14.7 | 12.1 | - | - |
| Volume swelling rate per superabsorbent polymer bead in case of 500 ppm addition (times) | 25.0 | 23.4 | 24.4 | 20.7 |
| Volume swelling rate per superabsorbent polymer bead in case of 1000 ppm addition (times) | 25.6 | 24.3 | 24.0 | 21.6 |

(continued)

| | Examples | | | |
|---|---|---|---|---|
| | 24 | 25 | 26 | 27 |
| Volume swelling rate per superabsorbent polymer bead in case of 2000 ppm addition (times) | 26.2 | 23.9 | 23.4 | 22.3 |
| Particle diameter of aggregate in case of 100 ppm addition (μm) | 6 | 6 | 59 | 6 |
| Particle diameter of aggregate in case of 200 ppm addition (μm) | 8 | 7 | - | - |
| Particle diameter of aggregate in case of 500 ppm addition (μm) | 29 | 42 | 56 | 10 |
| Particle diameter of aggregate in case of 1000 ppm addition (μm) | 43 | 35 | 58 | 13 |
| Particle diameter of aggregate in case of 2000 ppm addition (μm) | 43 | 36 | 32 | 27 |
| Hardness of aggregate in case of 500 ppm addition (mPa) | 171 | - | - | - |
| Rate of adsorption to erythrocyte in case of 500 ppm addition (%) | - | - | - | - |
| Total permeation amount (g) | 6.4 | 3.6 | - | - |
| Volume swelling rate per SAP bead in case of using permeation aqueous (times) | 24.5 | 25.1 | - | - |

Table 8

| | Examples | | |
|---|---|---|---|
| | 28 | 29 | 30 |
| Monomer A | Diethyl sulphate salt of dimethylaminoethyl methacrylate | Diethyl sulphate salt of dimethylaminoethyl methacrylate | Methyl chloride salt of diallylmethylamine |

(continued)

| | Examples | | |
|---|---|---|---|
| | 28 | 29 | 30 |
| Monomer B | Mathacrylic acid | Sodium styrene sulphonate | Acrylamide |
| Copolymerisation molar ratio of monomer A/monnomer B | 60/40 | 60/40 | 24/76 |
| Quaternary ammonium salt polycondensate | - | - | - |
| Molecular weight | 160000 | 100000 | 120000 |
| Organic value | 168 | 204 | 86 |
| Inorganic value | 468 | 714 | 203.4 |
| IOB value | 2.79 | 3.50 | 2.37 |
| Streaming potential (μeq/L) | 3474 | 1706 | 5243 |
| Agglutination speed (mPa·s/s) | | - | 0.09 |
| Volume swelling rate per superabsorbent polymer bead in case of 100 ppm addition (times) | 13.8 | - | 20.4 |
| Volume swelling rate per superabsorbent polymer bead in case of 200 ppm addition (times) | - | - | 25.3 |
| Volume swelling rate per superabsorbent polymer bead in case of 500 ppm addition (times) | 13.6 | 13.0 | 27.2 |
| Volume swelling rate per superabsorbent polymer bead in case of 1000 ppm addition (times) | 14.6 | 13.3 | 22.8 |
| Volume swelling rate per superabsorbent polymer bead in case of 2000 ppm addition (times) | 16.3 | 19.9 | - |
| Particle diameter of aggregate in case of 100 ppm addition (μm) | 7 | 7 | 5 |
| Particle diameter of aggregate in case of 200 ppm addition (μm) | - | - | 7 |
| Particle diameter of aggregate in case of 500 ppm addition (μm) | 6 | 7 | 11 |
| Particle diameter of aggregate in case of 1000 ppm addition (μm) | 7 | 5 | 10 |

(continued)

| | Examples | | |
|---|---|---|---|
| | 28 | 29 | 30 |
| Particle diameter of aggregate in case of 2000 ppm addition (μm) | 6 | 9 | - |
| Hardness of aggregate in case of 500 ppm addition (mPa) | 1 | - | - |
| Rate of adsorption to erythrocyte in case of 500 ppm addition (%) | - | - | - |
| Total permeation amount (g) | 7.9 | - | 7.7 |
| Volume swelling rate per SAP bead in case of using permeation aqueous (times) | 15.3 | - | 24.7 |

Table 9

| | Examples | | |
|---|---|---|---|
| | 31 | 32 | 33 |
| Monomer A | Diethyl sulphate salt of dimethylaminoethyl methacrylate | Diethyl sulphate salt of dimethylaminoethyl methacrylate | 3-dimethylamino-propylacrylamide |
| Monomer B | Butyl methacrylate | Styrene | 2-Hydroxyethyl methacrylate |
| Copolymerisation molar ratio of monomer A/monomer B | 65/35 | 57/43 | 96/4 |
| Quaternary ammonium salt polycondensate | - | - | |
| Molecular weight | 100000 | 100000 | 30000 |
| Organic value | 205 | 201.3 | 148 |
| Inorganic value | 463 | 394.05 | 265.6 |
| IOB value | 2.26 | 1.96 | 1.79 |
| Streaming potential (μeq/L) | 3737 | 3325 | 3214 |
| Agglutination speed (mPa·s/s) | 2.13 | - | - |
| Volume swelling rate per superabsorbent polymer bead in case of 100 ppm addition (times) | 11.7 | 13.4 | 14.7 |
| Volume swelling rate per superabsorbent polymer bead in case of 200 ppm addition (times) | 11.4 | 13.2 | - |
| Volume swelling rate per superabsorbent polymer bead in case of 500 ppm addition (times) | 24.6 | 21.8 | 24.9 |
| Volume swelling rate per superabsorbent polymer bead in case of 1000 ppm addition (times) | 28.0 | 25.5 | 20.7 |

(continued)

| | Examples | | |
|---|---|---|---|
| | 31 | 32 | 33 |
| Volume swelling rate per superabsorbent polymer bead in case of 2000 ppm addition (times) | 27.2 | 24.1 | 24.6 |
| Particle diameter of aggregate in case of 100 ppm addition (μm) | 7 | 6 | 9 |
| Particle diameter of aggregate in case of 200 ppm addition (μm) | 7 | 7 | - |
| Particle diameter of aggregate in case of 500 ppm addition (μm) | 29 | 31 | 24 |
| Particle diameter of aggregate in case of 1000 ppm addition (μm) | 21 | 61 | 33 |
| Particle diameter of aggregate in case of 2000 ppm addition (μm) | 32 | 62 | 45 |
| Hardness of aggregate in case of 500 ppm addition (mPa) | 232 | - | - |
| Rate of adsorption to erythrocyte in case of 500 ppm addition (%) | - | - | - |
| Total permeation amount (g) | 2.0 | 5.1 | - |
| Volume swelling rate per SAP bead in case of using permeation aqueous (times) | 17.4 | 16.7 | - |

Table 10

| | Examples | | | | |
|---|---|---|---|---|---|
| | 34 | 35 | 36 | 37 | 38 |
| Monomer A | Amidine | Ethyleneimine | Diallylamine | - | Diallylamine hydrochloride |
| Monomer B | - | - | - | - | - |
| Copolymerisation molar ratio of monomer A/monomer B | 100/0 | 100/0 | 100/0 | - | 100/0 |
| Quaternary ammonium salt polycondensate | - | - | - | Polycondensate of dimethylamine and epichlorohydrin | - |
| Molecular weight | 3000000 | 70000 | 5000 | 300000 | 50000 |
| Organic value | 140 | 40 | 120 | 130 | 160 |
| Inorganic value | 490 | 70 | 80 | 510 | 420 |
| IOB value | 3.50 | 1.75 | 0.67 | 3.92 | 2.63 |
| Streaming potential (μeq/L) | 5885 | 2742 | 4688 | 6849 | 7422 |

(continued)

| | Examples | | | | |
|---|---|---|---|---|---|
| | 34 | 35 | 36 | 37 | 38 |
| Agglutination speed (mPa·s/s) | - | 0.02 | - | - | |
| Volume swelling rate per superabsorbent polymer bead in case of 100 ppm addition (times) | - | 12.5 | - | - | 14.1 |
| Volume swelling rate per superabsorbent polymer bead in case of 200 ppm addition (times) | - | - | - | - | - |
| Volume swelling rate per superabsorbent polymer bead in case of 500 ppm addition (times) | 24.2 | 12.7 | 14.1 | 15.4 | 23.2 |
| Volume swelling rate per superabsorbent polymer bead in case of 1000 ppm addition (times) | 25.0 | 24.7 | 23.2 | 22.7 | 21.9 |
| Volume swelling rate per superabsorbent polymer bead in case of 2000 ppm addition (times) | Unmeasurable | 28.3 | 21.9 | 23.5 | 24.7 |
| Particle diameter of aggregate in case of 100 ppm addition (μm) | - | 7 | - | - | - |
| Particle diameter of aggregate in case of 200 ppm addition (μm) | - | - | - | - | - |
| Particle diameter of aggregate in case of 500 ppm addition (μm) | 19 | 14 | 36 | 14 | 66 |
| Particle diameter of aggegate in case of 1000 ppm addition (μm) | 21 | 44 | 39 | 22 | 81 |
| Particle diameter of aggregate in case of 2000 ppm addition (μm) | Unmeasurable | 65 | 47 | 39 | 97 |
| Hardness of aggregate in case of 500 ppm addition (mPa) | - | - | - | - | - |
| Rate of absorption to erythrocyte in case of 500 ppm addition (%) | - | - | - | - | - |
| Total permeation amount (g) | - | 8.0 | - | - | - |

(continued)

| | Examples | | | | |
|---|---|---|---|---|---|
| | 34 | 35 | 36 | 37 | 38 |
| Volume swelling rate per SAP bead in case of using permeation aqueous (times) | - | 12.7 | - | - | - |

Table 11

| | Examples | | | | | | |
|---|---|---|---|---|---|---|---|
| | 39 | 40 | 41 | 42 | 43 | 44 | 45 |
| Monomer A | Allylamine hydrochloride | Diallylamine hydrochloride | Ethyleneimine | - | Diethyl sulphate salt of dimethylaminoethyl methacrylate | Diethyl sulphate salt of diallylmethylamine | Diethyl sulphate salt of dimethylaminoethyl methacrylate |
| Monomer B | - | Acrylamide | - | - | Polyethylene glycol monomethyl methacrylate | - | Dimethylacrylamide |
| Copolymerisation molar ratio of monomer A/monomer B | 100/0 | 97/3 | 100/0 | - | 96/4 | 100/0 | 40/60 |
| Quaternary ammonium salt polycondensate | - | - | - | Polycondensate of dicyndiamide and diethylenetriamine | - | - | - |
| Molecular weight | 5000 | 20000 | 10000 | 3000 | 174000 | 37000 | 59000 |
| Organic value | 90 | 156.7 | 40 | 120 | 306 | 240 | 150 |
| Inorganic value | 410 | 411.45 | 70 | 410 | 790 | 630 | 353 |
| IOB value | 4.56 | 2.63 | 1.75 | 3.42 | 2.59 | 2.63 | 2.35 |
| Streaming potential (μeq/L) | 9407 | 8485 | 3790 | 5619 | 3568 | 4896 | 3861 |
| Agglutination speed (mPa·s/s) | 0.02 | | - | - | 0.15 | 0.12 | 0.13 |
| Volume swelling rate per superabsorbent polymer bead in case of 100 ppm addition (times) | - | 13.4 | 14.0 | 11.5 | 12.8 | 14.0 | 12.7 |
| Volume swelling rate per superabsorbent polymer bead in case of 200 ppm addition (times) | - | - | - | - | 13.2 | 19.7 | 18.7 |

(continued)

| | Examples | | | | | | |
|---|---|---|---|---|---|---|---|
| | 39 | 40 | 41 | 42 | 43 | 44 | 45 |
| Volume swelling rate per superabsorbent polymer bead in case of 500 ppm addition (times) | 15.7 | 18.2 | 13.9 | 13.0 | 21.4 | 28.4 | 23.0 |
| Volume swelling rate per superabsorbent polymer bead in case of 1000 ppm addition (times) | 21.7 | 19.4 | 16.2 | 12.2 | 20.4 | 25.9 | 24.4 |
| Volume swelling rate per superabsorbent polymer bead in case of 2000 ppm addition (times) | 24.2 | 22.9 | 22.1 | 22.2 | - | - | - |
| Particle diameter of aggregate in case of 100 ppm addition (μm) | - | 8 | 7 | 7 | 6 | 6 | 6 |
| Particle diameter of aggregate in case of 200 ppm addition (μm) | - | - | - | - | 7 | 8 | 9 |
| Particle diameter of aggregate in case of 500 ppm addition (μm) | 8 | 26 | 6 | 7 | 12 | 25 | 13 |
| Particle diameter of aggregate in case of 1000 ppm addition (μm) | 13 | 57 | 20 | 7 | 9 | - | 15 |
| Particle diameter of aggregate in case of 2000 ppm addition (μm) | 34 | 53 | 42 | 26 | - | - | - |
| Hardness of aggregate in case of 500 ppm addition (mPa) | - | - | - | - | 10 | - | - |

(continued)

| | Examples | | | | | | |
|---|---|---|---|---|---|---|---|
| | 39 | 40 | 41 | 42 | 43 | 44 | 45 |
| Rate of absorption to erythrocyte in case of 500 ppm addition (%) | - | - | - | - | - | - | - |
| Total permeation amount (g) | 7.2 | - | - | - | 7.7 | 7.2 | 7.7 |
| Volume swelling rate per SAP bead in case of using permeation aqueous (times) | 13.7 | - | - | - | 21.9 | 25.7 | 21.4 |

Table 12

| | Comparative Examples | | | |
|---|---|---|---|---|
| | 1 | 2 | 3 | |
| Monomer A | Diethyl sulphate salt of dimethylaminoethyl methacrylate | Ethyleneimine | - | |
| Monomer B | - | - | - | |
| Copolymerisation molar ratio of monomer A/monomer B | 100/0 | 100/0 | - | |
| Quaternary ammonium salt polycondensate | - | - | - | |
| Polymer structure | - | - | 1) | 1) 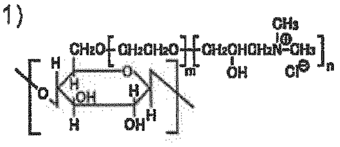 |
| Molecular weight | 311 | 1800 | 1112000 | |
| Organic value | 240 | 40 | - | |
| Inorganic value | 682 | 70 | - | |
| IOB value | 2.84 | 1.75 | - | |
| Streaming potential (μeq/L) | 29 | 364 | 618 | |
| Agglutination speed (mPa·s/s) | 0.00 | 0.00 | 0.00 | |
| Volume swelling rate per superabsorbent polymer bead in case of 100 ppm addition (times) | - | 11.5 | - | |
| Volume swelling rate per superabsorbent polymer bead in case of 200 ppm addition (times) | - | - | - | |
| Volume swelling rate per superabsorbent polymer bead in case of 500 ppm addition (times) | 12.6 | 15.3 | 13.2 | |
| Volume swelling rate per superabsorbent polymer bead in case of 1000 ppm addition (times) | 13.8 | 13.4 | 13.2 | |

(continued)

| | Comparative Examples | | |
|---|---|---|---|
| | 1 | 2 | 3 |
| Volume swelling rate per superabsorbent polymer bead in case of 2000 ppm addition (times) | 13.9 | | 13.9 |
| Particle diameter of aggregate in case of 100 ppm addition (μm) | - | 7 | - |
| Particle diameter of aggregate in case of 200 ppm addition (μm) | - | - | - |
| Particle diameter of aggregate in case of 500 ppm addition (μm) | 6 | 11 | 7 |
| Particle diameter of aggregate in case of 1000 ppm addition (μm) | 7 | 7 | 7 |
| Particle diameter of aggregate in case of 2000 ppm addition (μm) | 6 | 5 | 6 |
| Hardness of aggregate in case of 500 ppm addition (mPa) | - | - | - |
| Rate of absorption to erythrocyte in case of 500 ppm addition (%) | - | - | - |
| Total permeation amount (g) | 8.1 | 8.1 | 8.1 |
| Volume swelling rate per superabsorbent polymer bead in case of using permeation aqueous (times) | 12.0 | 12.0 | 12.0 |

As is clear from the results shown in Tables 2 to 12, it was found that, in the examples, the erythrocytes agglutinate as a result of using the quaternary ammonium salt polymer, and as a result, the superabsorbent polymer is not prevented from swelling, has a fast menstrual blood absorption speed and an increased absorption amount. In contrast, it is found that, in the comparative examples, the erythrocytes insufficiently agglutinate, and as a result, swelling

of the superabsorbent polymer is hindered and the superabsorbent polymer has a slow menstrual blood absorption speed and a reduced absorption amount.

Industrial Applicability

The sanitary product of the present invention includes a specific cationic polymer, and as a result, the menstrual blood absorption speed of the superabsorbent polymer is faster and the absorption amount is larger in the sanitary product of the present invention than in a sanitary product that includes the same superabsorbent polymer and does not include the cationic polymer. In addition, when a sanitary product is treated using the treatment agent for a sanitary product of the present invention, the sanitary product has a faster menstrual blood absorption speed and an increased absorption amount compared with a sanitary product that includes the same superabsorbent polymer.

## Claims

1. A sanitary product comprising a water-soluble cationic polymer that has a structure including a main chain and a side chain bound to the main chain and that has a molecular weight of 2000 or more, wherein the water-soluble cationic polymer is:

   a quaternary ammonium salt homopolymer having a repeating unit represented by Formula 1 below; or
   a quaternary ammonium salt copolymer having a repeating unit represented by Formula 1 below and a repeating unit represented by Formula 2 below, and

   the main chain and the side chain of the water-soluble cationic polymer are bound to each other at one point, and the side chain has a quaternary ammonium moiety.

[Chem. 1]

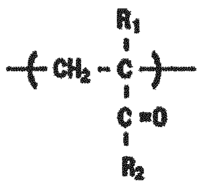

(1)

In the formula, $R_1$ represents H or $CH_3$.

$R_2$ represents 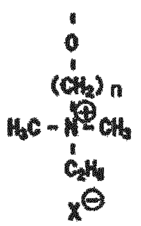 or 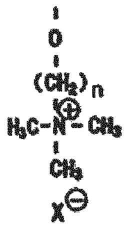 or 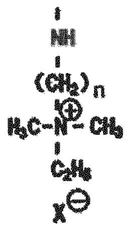 or 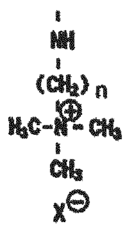 .

n represents an integer between 1 and 10.

$X^-$ represents a halide ion, $C_2H_5OSO_3^-$ or $CH_3OSO_3^-$.

[Chem. 2]

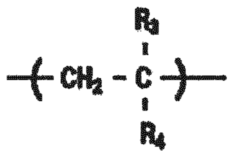

(2)

In the formula, $R_3$ represents H or $CH_3$.

$R_4$ represents 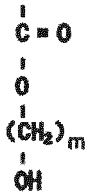 or 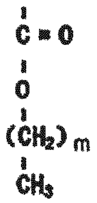 or 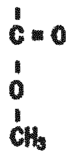 or 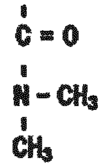 or 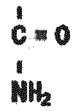 or

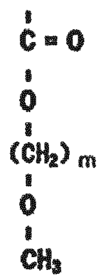 or 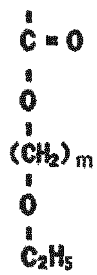 or 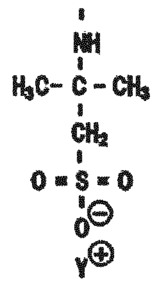 or 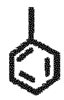

m represents an integer between 1 and 10.

$Y^+$ represents $Na^+$ or $K^+$.

2. The sanitary product according to claim 1, wherein the side chain has four or more and ten or less carbon atoms.

3. The sanitary product according to claim 2, wherein the side chain has five or more and nine or less carbon atoms.

4. The sanitary product according to any one of claims 1 to 3, wherein the quaternary ammonium salt copolymer is a copolymer of a polymerisable monomer having a quaternary ammonium moiety and a cationic polymerisable monomer or a nonionic polymerisable monomer.

5. The sanitary product according to claim 4, wherein the cationic polymerisable monomer is vinylpyridine or a condensation compound of dicyandiamide and diethylenetriamine.

6. The sanitary product according to claim 4, wherein an anionic polymerisable monomer is 2-acrylamide-2-methylpropane sulphonic acid, methacrylic acid, acrylic acid, styrene sulphonic acid, or a salt of these compounds.

7. The sanitary product according to claim 4, wherein the nonionic polymerisable monomer is vinyl alcohol, acrylamide, dimethylacrylamide, ethylene glycol, propylene glycol, ethylene glycol monomethacrylate, ethylene glycol monoacrylate, hydroxyethyl methacrylate, hydroxyethyl acrylate, methyl methacrylate, methyl acrylate, ethyl methacrylate, ethyl acrylate, propyl methacrylate, propyl acrylate, butyl methacrylate, or butyl acrylate.

8. The sanitary product according to any one of claims 1 to 3, wherein the quaternary ammonium salt copolymer is a copolymer of a polymerisable monomer having a quaternary ammonium moiety and a polymerisable monomer having a functional group capable of forming a hydrogen bond.

9. The sanitary product according to claim 8, wherein the functional group capable of forming a hydrogen bond is -OH,

$-NH_2$, -CHO, -COOH, -HF, or -SH.

**10.** The sanitary product according to claim 8 or 9, wherein the polymerisable monomer having a functional group capable of forming a hydrogen bond is hydroxyethyl methacrylate, vinyl alcohol, acrylamide, dimethyl acrylamide, ethylene glycol, propylene glycol, ethylene glycol monomethacrylate, ethylene glycol monoacrylate, hydroxyethyl methacrylate, or hydroxyethyl acrylate.

**11.** The sanitary product according to any one of claims 1 to 3, wherein the quaternary ammonium salt copolymer is a copolymer of a polymerisable monomer having a quaternary ammonium moiety and a polymerisable monomer having a functional group capable of making hydrophobic interaction.

**12.** The sanitary product according to claim 11, wherein the functional group capable of making hydrophobic interaction is an alkyl group, a phenyl group, an alkylnaphthalene group, or a fluorinated alkyl group.

**13.** The sanitary product according to claim 11 or 12, wherein the polymerisable monomer having a functional group capable of making hydrophobic interaction is methyl methacrylate, methyl acrylate, ethyl methacrylate, ethyl acrylate, propyl methacrylate, propyl acrylate, butyl methacrylate, butyl acrylate, or styrene.

**14.** A sanitary product to be used to absorb menstrual blood,
the sanitary product comprising a superabsorbent polymer,
wherein at least one member constituting the sanitary product comprises a water-soluble cationic polymer which includes a quaternary ammonium salt homopolymer or a quaternary ammonium salt copolymer,
the quaternary ammonium salt homopolymer and the quaternary ammonium salt copolymer has a streaming potential of 1500 μeq/L or more and a molecular weight of 2000 or more, and
an amount of the water-soluble cationic polymer in the sanitary product is from 0.2 $g/m^2$ to 20 $g/m^2$.

**15.** The sanitary product according to claim 14, wherein the quaternary ammonium salt homopolymer or the quaternary ammonium salt copolymer has a structure including a main chain and a side chain bound to the main chain, and the main chain and the side chain are bound to each other at one point.

**16.** The sanitary product according to claim 15, wherein the side chain has four or more and ten or less carbon atoms.

**17.** The sanitary product according to claim 16, wherein the side chain has five or more and nine or less carbon atoms.

**18.** The sanitary product according to any one of claims 14 to 17, wherein the quaternary ammonium salt is a salt of a quaternary ammonium cation, a neutralised salt of a tertiary amine, or a tertiary amine that becomes cationised in an aqueous solution.

**19.** The sanitary product according to any one of claims 14 to 18, wherein the quaternary ammonium salt copolymer is a copolymer obtained by copolymerising two or more polymerisable monomers and encompasses both binary copolymers and copolymers including three or more components.

**20.** The sanitary product according to any one of claims 14 to 19, wherein the quaternary ammonium salt copolymer is obtained by copolymerising two or more types of polymerisable monomers having a quaternary ammonium moiety, or copolymerising one or more types of polymerisable monomers having a quaternary ammonium moiety and one or more types of polymerisable monomers having no quaternary ammonium moiety.

**21.** The sanitary product according to any one of claims 14 to 20,
wherein the quaternary ammonium salt homopolymer has a repeating unit represented by Formula 1 below, and the quaternary ammonium salt copolymer has a repeating unit represented by Formula 1 below and a repeating unit represented by Formula 2 below

[Chem. 3]

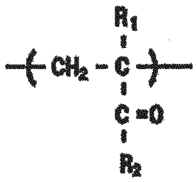

(1)

In the formula, $R_1$ represents H or $CH_3$.

$R_2$ represents 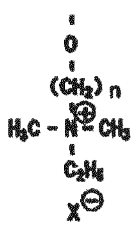 or 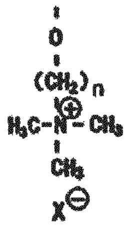 or 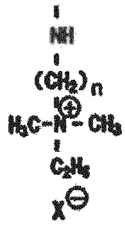 or 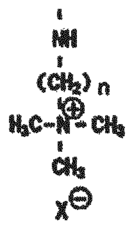 .

n represents an integer between 1 and 10.

$X^-$ represents a halide ion, $C_2H_5OSO_3^-$ or $CH_3OSO_3^-$.

[Chem. 4]

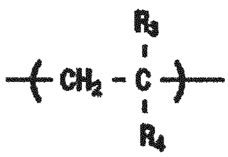

(2)

In the formula, $R_3$ represents H or $CH_3$.

$R_4$ represents 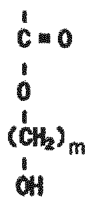 or 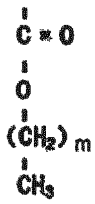 or 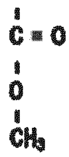 or 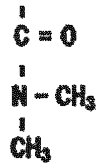 or 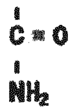 or

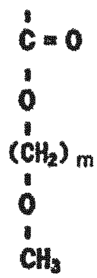 or 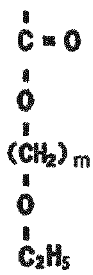 or 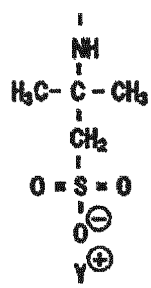 or 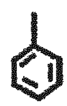 .

m represents an integer between 1 and 10.

$Y^+$ represents $Na^+$ or $K^+$.

**22.** The sanitary product according to any one of claims 15 to 21, wherein the quaternary ammonium salt homopolymer or the quaternary ammonium salt copolymer has a streaming potential of 13000 μeq/L or less.

**23.** The sanitary product according to any one of claims 14 to 22, wherein the quaternary ammonium salt homopolymer or the quaternary ammonium salt copolymer has a streaming potential of from 1500 μeq/L to 13000 μeq/L.

**24.** The sanitary product according to any one of claims 14 to 23,
wherein the quaternary ammonium salt homopolymer has a molecular weight of ten million or less, or
the quaternary ammonium salt copolymer is a copolymer of a polymerisable monomer having a quaternary ammonium moiety and a cationic polymerisable monomer, an anionic polymerisable monomer, or a nonionic polymerisable monomer, and has a molecular weight of ten million or less.

**25.** The sanitary product according to claim 24, wherein the cationic polymerisable monomer is vinylpyridine or a condensation compound of dicyandiamide and diethylenetriamine.

**26.** The sanitary product according to claim 24, wherein the anionic polymerisable monomer is 2-acrylamide-2-methylpropane sulphonic acid, methacrylic acid, acrylic acid, styrene sulphonic acid, or a salt of these compounds.

**27.** The sanitary product according to claim 24, wherein the nonionic polymerisable monomer is vinyl alcohol, acrylamide, dimethylacrylamide, ethylene glycol, propylene glycol, ethylene glycol monomethacrylate, ethylene glycol monoacrylate, hydroxyethyl methacrylate, hydroxyethyl acrylate, methyl methacrylate, methyl acrylate, ethyl methacrylate, ethyl acrylate, propyl methacrylate, propyl acrylate, butyl methacrylate, or butyl acrylate.

**28.** A sanitary product that is an absorbent article comprising a superabsorbent polymer, a constituent member located closer to a skin of a wearer than the superabsorbent polymer, and a water-soluble cationic polymer,
the water-soluble cationic polymer being arranged in a portion of the absorbent article located closer to the skin than the constituent member, and having an agglutination speed of 0.75 mPa·s/s or less.

**29.** The sanitary product according to claim 28, wherein the water-soluble cationic polymer has an agglutination speed of 0.32 mPa·s/s or less.

**30.** The sanitary product according to claim 28 or 29, wherein the water-soluble cationic polymer is a quaternary ammonium salt homopolymer or a quaternary ammonium salt copolymer.

**31.** The sanitary product according to any one of claims 28 to 30,
wherein the quaternary ammonium salt homopolymer has a molecular weight of ten million or less, or
the quaternary ammonium salt copolymer is a copolymer of a polymerisable monomer having a quaternary ammonium moiety and a cationic polymerisable monomer, an anionic polymerisable monomer, or a nonionic polymerisable monomer, and has a molecular weight of ten million or less.

**32.** The sanitary product according to claim 31, wherein the cationic polymerisable monomer is vinylpyridine or a condensation compound of dicyandiamide and diethylenetriamine.

**33.** The sanitary product according to claim 31, wherein the anionic polymerisable monomer is 2-acrylamide-2-methylpropane sulphonic acid, methacrylic acid, acrylic acid, styrene sulphonic acid, or a salt of these compounds.

**34.** The sanitary product according to claim 31, wherein the nonionic polymerisable monomer is vinyl alcohol, acrylamide, dimethylacrylamide, ethylene glycol, propylene glycol, ethylene glycol monomethacrylate, ethylene glycol monoacrylate, hydroxyethyl methacrylate, hydroxyethyl acrylate, methyl methacrylate, methyl acrylate, ethyl methacrylate, ethyl acrylate, propyl methacrylate, propyl acrylate, butyl methacrylate, or butyl acrylate.

**35.** The sanitary product according to any one of claims 28 to 34, wherein the quaternary ammonium salt copolymer is a copolymer of a polymerisable monomer having a quaternary ammonium moiety and a cationic polymerisable monomer or a nonionic polymerisable monomer.

**36.** The sanitary product according to any one of claims 28 to 35, wherein the quaternary ammonium salt homopolymer or a quaternary ammonium salt copolymer is a quaternary ammonium formed using an alkylating agent.

**37.** The sanitary product according to claim 36, wherein the alkylating agent is a dialkyl sulphate ester.

**38.** The sanitary product according to any one of claims 28 to 37, wherein the quaternary ammonium salt homopolymer or the quaternary ammonium salt copolymer is a quaternary ammonium formed using dialkyl sulphate.

**39.** The sanitary product according to any one of claims 28 to 38, wherein the quaternary ammonium salt homopolymer or the quaternary ammonium salt copolymer has a molecular weight of 2000 or more and three million or less.

**40.** The sanitary product according to any one of claims 28 to 39, wherein the quaternary ammonium salt copolymer is a copolymer of a polymerisable monomer having a quaternary ammonium moiety and a polymerisable monomer having a functional group capable of forming a hydrogen bond.

**41.** The sanitary product according to claim 40, wherein the functional group capable of forming a hydrogen bond is -OH, $-NH_2$, -CHO, -COOH, -HF, or -SH.

**42.** The sanitary product according to claim 40 or 41, wherein the polymerisable monomer having a functional group capable of forming a hydrogen bond is hydroxyethyl methacrylate, vinyl alcohol, acrylamide, dimethyl acrylamide, ethylene glycol, propylene glycol, ethylene glycol monomethacrylate, ethylene glycol monoacrylate, hydroxyethyl methacrylate, or hydroxyethyl acrylate.

**43.** The sanitary product according to any one of claims 28 to 38, wherein the quaternary ammonium salt copolymer is a copolymer of a polymerisable monomer having a quaternary ammonium moiety and a polymerisable monomer having a functional group capable of making hydrophobic interaction.

**44.** The sanitary product according to claim 43, wherein the functional group capable of making hydrophobic interaction is an alkyl group, a phenyl group, an alkylnaphthalene group, or a fluorinated alkyl group.

**45.** The sanitary product according to claim 43 or 44, wherein the polymerisable monomer having a functional group capable of making hydrophobic interaction is methyl methacrylate, methyl acrylate, ethyl methacrylate, ethyl acrylate, propyl methacrylate, propyl acrylate, butyl methacrylate, butyl acrylate, or styrene.

**46.** The sanitary product according to any one of claims 28 to 45, wherein the quaternary ammonium salt copolymer is a copolymer of a polymerisable monomer having a quaternary ammonium moiety and a polymerisable monomer having no quaternary ammonium moiety, and a molar ratio of the polymerisable monomer having a quaternary ammonium moiety in the copolymer is 70 mol% or more.

**47.** The sanitary product according to any one of claims 28 to 46, wherein the quaternary ammonium salt homopolymer or a quaternary ammonium salt copolymer is obtained by a homopolymerisation method or a copolymerisation method using a vinyl-based polymerisable monomer, and
the polymerisation method is radical polymerisation, living radical polymerisation, living cationic polymerisation, living anionic polymerisation, coordinated polymerisation, ring-opening polymerisation, or polycondensation.

**48.** The sanitary product according to any one of claims 1 to 47, comprising the water-soluble cationic polymer in an amount of from 0.1 mass% to 10 mass%.

**49.** The sanitary product according to any one of claims 1 to 48, wherein a treatment agent containing a quaternary ammonium salt homopolymer, a quaternary ammonium salt copolymer, or a quaternary ammonium salt polycondensate is applied to the sanitary product, and the treatment agent further contains a solvent, a plasticiser, an aromatic agent, an antibacterial and deodorising agent, and a skin care agent.

**50.** The sanitary product according to claim 49, wherein water, a water-soluble organic solvent, or a mixed solvent of the water-soluble organic solvent and water is used as the solvent.

**51.** The sanitary product according to claim 49 or 50, wherein glycerin, polyethylene glycol, propylene glycol, ethylene glycol, or 1,3-butanediol is used as the plasticiser.

**52.** The sanitary product according to any one of claims 49 to 51, wherein an aromatic agent having a green herbal aroma, a plant extract, or a citrus extract is used as the aromatic agent.

**53.** The sanitary product according to any one of claims 49 to 52, wherein a cancrinite-like mineral containing a metal having an antibacterial property, a porous polymer obtained polymerising a polymerisable monomer having a phenyl group, a quaternary ammonium salt, active carbon, or a clay mineral is used as the antibacterial and deodorising agent.

**54.** The sanitary product according to any one of claims 49 to 53, wherein a plant extract, collagen, a natural moisturising component, a moisturising agent, a keratin softening agent, or an antiphilogistic is used as the skin care agent.

**55.** The sanitary product according to any one of claims 1 to 54, wherein the sanitary product comprises the absorbent member which includes a covering sheet, a topsheet, and a second sheet arranged between the covering sheet and the topsheet, and
a quaternary ammonium salt homopolymer, a quaternary ammonium salt copolymer, or a quaternary ammonium salt polycondensate is applied to at least one of the covering sheet, the topsheet and the second sheet.

**56.** The sanitary product according to any one of claims 1 to 55, wherein when blood is absorbed, an aggregate of erythrocytes is formed.

**57.** The sanitary product according to any one of claims 1 to 56, which is a sanitary towel, a pantyliner, or a tampon.

**58.** A treatment agent for a sanitary product to be used to treat a constituent member other than a superabsorbent polymer of a sanitary product that contains the superabsorbent polymer and is to be used to absorb menstrual blood, wherein the treatment agent contains a water-soluble cationic polymer having a molecular weight of 2000 or more, the cationic polymer has a value of inorganic value/organic value, which expresses a ratio between an inorganic value and an organic value, of from 0.6 to 4.6, and
the cationic polymer is a quaternary ammonium salt homopolymer, a quaternary ammonium salt copolymer, or a quaternary ammonium salt polycondensate.

**59.** The treatment agent for a sanitary product according to claim 58, wherein the cationic polymer has an IOB value of from 0.6 to 4.6.

**60.** The treatment agent for a sanitary product according to claim 58 or 59, wherein the quaternary ammonium salt homopolymer or the quaternary ammonium salt copolymer is a quaternary ammonium formed using dialkyl sulphate, is formed by neutralisation by being dissolved in acid or water, or is a quaternary ammonium formed through a nucleophilic reaction including a condensation reaction.

**61.** The treatment agent for a sanitary product according to any one of claims 58 to 60,
wherein the quaternary ammonium salt homopolymer, the quaternary ammonium salt copolymer, or the quaternary ammonium salt polycondensate is formed by neutralisation of a tertiary amine using acid, and
hydrochloric acid, sulphuric acid, nitric acid, acetic acid, citric acid, phosphoric acid, fluorosulphonic acid, boric acid, chromic acid, lactic acid, oxalic acid, tartaric acid, gluconic acid, formic acid, ascorbic acid, or hyaluronic acid is used as the acid.

**62.** The treatment agent for a sanitary product according to any one of claims 58 to 61, wherein the value of inorganic value/organic value is from 1.8 to 3.6.

**63.** The treatment agent for a sanitary product according to any one of claims 58 to 62, wherein the value of inorganic value/organic value is from 2.2 to 3.0.

**64.** The treatment agent for a sanitary product according to any one of claims 58 to 63,
wherein the cationic polymer is a quaternary ammonium salt copolymer, and
the quaternary ammonium salt copolymer is a copolymer of a polymerisable monomer having a quaternary ammonium moiety and a polymerisable monomer having no quaternary ammonium moiety, and a molar ratio of the polymerisable monomer having a quaternary ammonium moiety in the copolymer is 10 mol% or more.

**65.** The treatment agent for a sanitary product according to any one of claims 58 to 64, wherein the cationic polymer is a quaternary ammonium salt copolymer, and the quaternary ammonium salt copolymer is a copolymer of a polymerisable monomer having a quaternary ammonium moiety and a cationic polymerisable monomer or a nonionic polymerisable monomer.

**66.** The treatment agent for a sanitary product according to any one of claims 58 to 65, wherein the cationic polymer has an organic value of from 100 to 240.

**67.** The treatment agent for a sanitary product according to any one of claims 58 to 66, wherein the cationic polymer has an organic value of from 130 to 190.

**68.** The treatment agent for a sanitary product according to any one of claims 58 to 67, wherein the cationic polymer has an inorganic value of from 90 to 680.

**69.** The treatment agent for a sanitary product according to any one of claims 58 to 68, wherein the cationic polymer has an inorganic value of from 250 to 490.

**70.** The treatment agent for a sanitary product according to any one of claims 58 to 69, wherein when x represents the organic value of the cationic polymer, and y represents the inorganic value of the cationic polymer, x and y satisfy a relationship y=ax (a is from 0.66 to 4.56).

**71.** The treatment agent for a sanitary product according to any one of claims 58 to 70, wherein the sanitary product comprises a topsheet, and the treatment agent is used to treat the topsheet.

**72.** The treatment agent for a sanitary product according to any one of claims 58 to 71, wherein the sanitary product comprises an absorbent core and a covering sheet that covers the absorbent core, and the treatment agent is used to treat the covering sheet.

**73.** The treatment agent for a sanitary product according to any one of claims 58 to 72, wherein the sanitary product comprises a topsheet, an absorbent member and a second sheet located between the topsheet and the absorbent member, and the treatment agent is used to treat the second sheet.

**74.** A sanitary product to which the treatment agent for a sanitary product according to any one of claims 58 to 73 is applied.

**75.** The sanitary product according to claim 74, wherein, when blood is absorbed, an aggregate of erythrocytes is formed.

**76.** The sanitary product according to claim 74 or 75, which is a sanitary towel, a pantyliner, or a tampon.

**77.** Use of a water-soluble cationic polymer as a blood agglutinating agent,
wherein the water-soluble cationic polymer has a structure including a main chain and a side chain bound to the main chain and has a molecular weight of 2000 or more,
the water-soluble cationic polymer is:

a quaternary ammonium salt homopolymer having a repeating unit represented by Formula 1 below; and
a quaternary ammonium salt copolymer having a repeating unit represented by Formula 1 below and a repeating unit represented by Formula 2 below, and

the main chain and the side chain are bound to each other at one point, and the side chain has a quaternary ammonium moiety.

[Chem. 5]

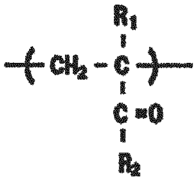 (1)

In the formula, $R_1$ represents H or $CH_3$.

$R_2$ represents 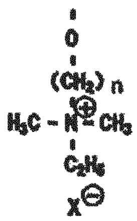 or 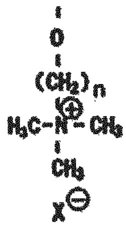 or 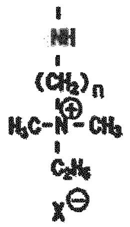 or 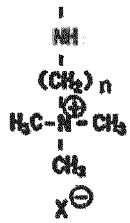 .

n represents an integer between 1 and 10.

$X^-$ represents a halide ion, $C_2H_5OSO_3^-$ or $CH_3OSO_3^-$.

[Chem. 6]

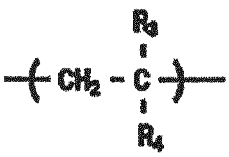 (2)

In the formula, $R_3$ represents H or $CH_3$.

$R_4$ represents 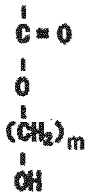 or 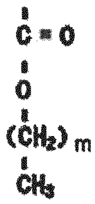 or 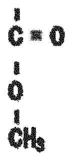 or 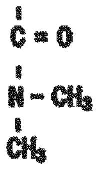 or 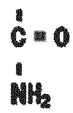 or

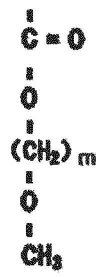 or 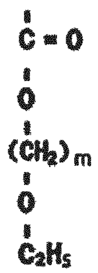 or 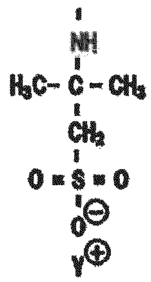 or 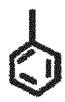 .

m represents an integer between 1 and 10.

$Y^+$ represents $Na^+$ or $K^+$.

**78.** Use of a water-soluble cationic polymer as a blood agglutinating agent, the water-soluble cationic polymer being a quaternary ammonium salt homopolymer or a quaternary ammonium salt copolymer which has a streaming potential of 1500 $\mu$eq/L or more and a molecular weight of 2000 or more.

**79.** Use of a water-soluble cationic polymer as a blood agglutinating agent, the water-soluble cationic polymer having an agglutination speed of 0.75 mPa·s/s or less.

**80.** Use of a water-soluble cationic polymer as a blood agglutinating agent,
wherein the water-soluble cationic polymer has a molecular weight of 2000 or more,
the water-soluble cationic polymer has a value of inorganic value/organic value, which expresses a ratio between an inorganic value and an organic value, of from 0.6 to 4.6, and
the water-soluble cationic polymer is a quaternary ammonium salt homopolymer, a quaternary ammonium salt copolymer, or a quaternary ammonium salt polycondensate.

Fig. 1

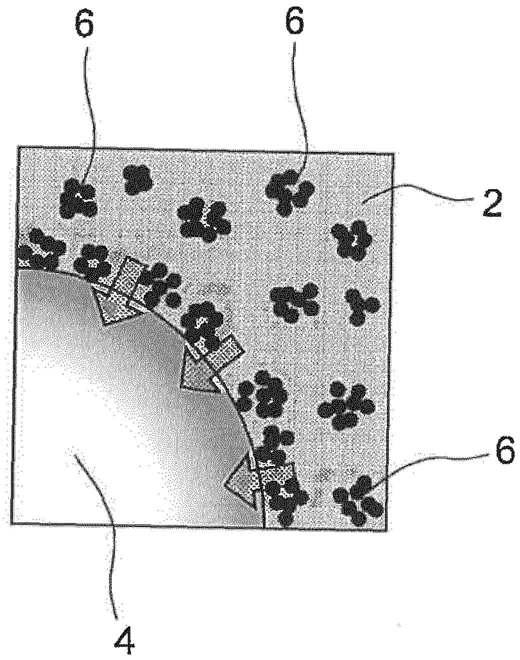


Fig. 2(a)

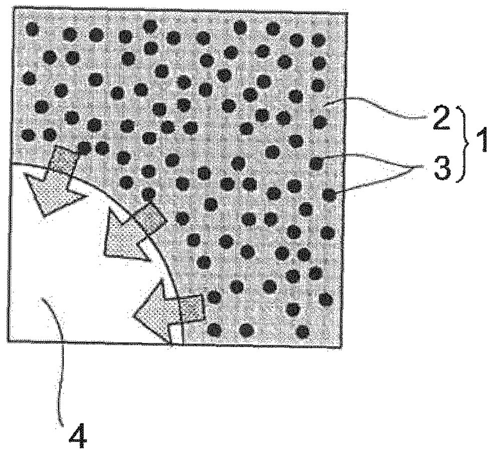


Fig. 2(b)

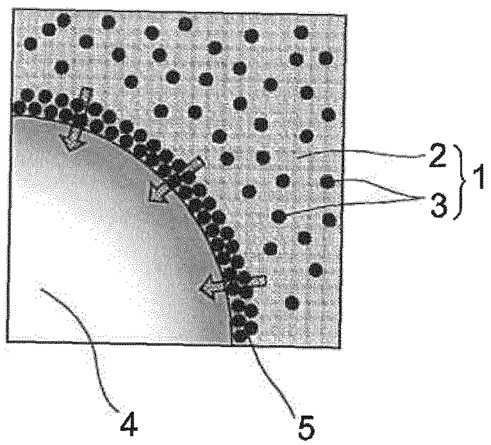

## INTERNATIONAL SEARCH REPORT

International application No.
PCT/JP2015/084407

A. CLASSIFICATION OF SUBJECT MATTER
*A61L15/48*(2006.01)i, *A61F13/472*(2006.01)i, *A61F13/20*(2006.01)i, *A61F13/53*(2006.01)i, *C08F20/34*(2006.01)i

According to International Patent Classification (IPC) or to both national classification and IPC

B. FIELDS SEARCHED

Minimum documentation searched (classification system followed by classification symbols)
A61L15/48, A61F13/00, A61F13/15-13/84, C08F20/34

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2002/059214 A1 (Nippon Shokubai Co., Ltd.), 01 August 2002 (01.08.2002), description, page 20, line 3 to page 24, line 23; page 36, line 18 to page 38, line 10 | 1-4,7-11, 21-24,27<br>77<br>5-6,12-13, 25-26 |
| X<br>Y<br>A | JP 2009-506056 A (Quick-Med Technologies, Inc., University of Florida Research Foundation, Inc.), 12 February 2009 (12.02.2009), paragraphs [0024] to [0061], [0078] | 1-3,21-24<br>77<br>4-13,25-27 |
| X<br>Y | JP 2003-519245 A (Kimberly-Clark Worldwide, Inc.), 17 June 2003 (17.06.2003), claim 19; paragraph [0044] | 78-79<br>77 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 March 2016 (04.03.16) | 15 March 2016 (15.03.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2015/084407

C (Continuation). DOCUMENTS CONSIDERED TO BE RELEVANT

| Category* | Citation of document, with indication, where appropriate, of the relevant passages | Relevant to claim No. |
|---|---|---|
| Y | JP 7-5173 A (Enzymatics, Inc.), 10 January 1995 (10.01.1995), paragraphs [0023] to [0029] | 77 |
| A | JP 9-509343 A (The Procter & Gamble Co.), 22 September 1997 (22.09.1997), page 7, line 1 to page 11, line 1; page 12, line 8 to page 13, line 9; page 36, lines 1 to 10 | 1-13,21-27, 77-79 |
| A | JP 2-140147 A (Syntex (U.S.A.) Inc.), 29 May 1990 (29.05.1990), claim 2; column 25, line 20 to column 29, line 2; column 32, line 18 to column 34, line 7 | 1-13,21-27, 77-79 |
| A | JP 2002-35580 A (Sanyo Chemical Industries, Ltd.), 05 February 2002 (05.02.2002), paragraphs [0016], [0019], [0023] | 1-13,21-27, 77-79 |
| A | US 2010/0174260 A1 (KIMBERLY-CLARK WORLDWIDE, INC.), 08 July 2010 (08.07.2010), specification, paragraphs [0011], [0039], [0058], [0061] | 1-13,21-27, 77-79 |
| A | JP 2002-528232 A (Kimberly-Clark Worldwide, Inc.), 03 September 2002 (03.09.2002), claims 5, 36; paragraph [0022] | 1-13,21-27, 77-79 |

Form PCT/ISA/210 (continuation of second sheet) (January 2015)

**INTERNATIONAL SEARCH REPORT**
Information on patent family members

International application No.
PCT/JP2015/084407

| | | |
|---|---|---|
| WO 2002/059214 A1 | 2002.08.01 | JP 2003-62460 A<br>JP 3987348 B2<br>US 2003/0069359 A1<br>specification, paragraphs [0146] to [0160], [0239] to [0248]<br>US 2006/0229413 A1<br>US 7495056 B2<br>EP 1364992 A1<br>BR 0203825 A<br>CN 1455802 A<br>CN 1262604 C |
| JP 2009-506056 A | 2009.02.12 | WO 2007/024974 A2<br>description, page 9, line 18 to page 18, line 2; page 24, lines 5 to 14<br>US 2009/0181157 A1<br>US 7790217 B2<br>US 2010/0330261 A1<br>US 8092854 B2<br>US 6387495 B1<br>EP 1937418 A2<br>CA 2620203 A1<br>CA 2620203 C<br>CN 101291743 A<br>CN 101291743 B<br>ZA 200801601 A<br>AU 2006283043 A1<br>AU 2006283043 B2<br>BR PI0617099-4 A2 |
| JP 2003-519245 A | 2003.06.17 | WO 2000/062825 A2<br>claim 19; description, page 21, line 12 to page 22, line 2<br>US 6387495 B1<br>EP 1171171 A2<br>EP 1171171 B1<br>DE 60022871 T2<br>AU 4356500 A<br>AU 760265 B2<br>BR 0009808 A<br>CZ 2001-3564 A3<br>CA 2362314 A1<br>PL 364857 A1<br>AT 305314 T<br>TW I231761 B<br>CN 1348388 A<br>CN 1187099 C<br>KR 20020013850 A<br>MX PA01010256 A<br>MX 238612 B<br>RU 2248221 C2<br>TR 200102986 T2 |

Form PCT/ISA/210 (patent family annex) (January 2015)

**INTERNATIONAL SEARCH REPORT**
Information on patent family members

International application No.
PCT/JP2015/084407

| | | |
|---|---|---|
| | | CO 5160300 A1<br>AR 042371 A2<br>AR 039044 A1<br>PE 14082000 A1<br>PH 12000000949 B1<br>IN 1371CH2001 A |
| JP 7-5173 A | 1995.01.10 | EP 0597577 A1<br>column 4, line 55 to column 6, line 27<br>CA 2104976 A1 |
| JP 9-509343 A | 1997.09.22 | WO 1995/022355 A1<br>description, page 4, line 5 to page 7, line 35; page 9, lines 1 to 32; page 31, lines 23 to 34<br>US 5851672 A<br>EP 0744965 A1<br>AU 1691795 A<br>BR PI9506831-7 A<br>CA 2181695 C<br>SG 55132 A1<br>CN 1141005 A<br>MX 9603474 A<br>KR 100367450 B1<br>PE 47595 A1<br>IN 191004 B |
| JP 2-140147 A | 1990.05.29 | EP 0325413 A2<br>claim 2; column 8, line 28 to column 9, line 27; column 10, line 43 to column 11, line 14<br>CA 1318588 C |
| JP 2002-35580 A | 2002.02.05 | (Family: none) |
| US 2010/0174260 A1 | 2010.07.08 | US 2002/0040210 A1<br>US 7687681 B2<br>GB 2379394 A<br>GB 2379394 B<br>WO 2001/091684 A2<br>DE 10196245 T1<br>AU 7490701 A<br>BR PI0111194-9 A<br>KR 20030068037 A<br>KR 10-0839714 B1<br>AR 028629 A1<br>MX PA02011670 A |
| JP 2002-528232 A | 2002.09.03 | WO 2000/025835 A1<br>claims 5, 36; description, page 9, lines 12 to 27 |

Form PCT/ISA/210 (patent family annex) (January 2015)

**INTERNATIONAL SEARCH REPORT**
Information on patent family members

International application No.
PCT/JP2015/084407

US 6350711 B1
US 2002/0065495 A1
US 6812169 B2
EP 1124586 A1
AU 1333900 A
AU 200013339 B2
BR 9914938 A
TW 476637 B
CN 1325314 A
CN 1208092 C
CO 5100934 A1
RU 2001114502 A
AR 021857 A1

Form PCT/ISA/210 (patent family annex) (January 2015)

**INTERNATIONAL SEARCH REPORT**

International application No.
PCT/JP2015/084407

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
1-13, 21-27 and 77-79

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.
PCT/JP2015/084407

Continuation of Box No.III of continuation of first sheet(2)

(Invention 1) claims 1-13, 21-27 and 77-79

Claims 1-13, 21-27, and 77 have a special technical feature wherein a water-soluble cationic polymer has been applied, the polymer being constituted of a structure which comprises a main chain and a side chain bonded thereto and having a molecular weight of 2,000 or higher, the polymer being either a quaternary-ammonium-salt homopolymer having repeating units represented by formula 1 or a quaternary-ammonium-salt copolymer having repeating units represented by formula 1 and repeating units represented by formula 2, the main chain and side chain of the water-soluble cationic polymer having been bonded at one site and the side chain having a quaternary ammonium moiety.

Claims 78-79 have a relationship such that these claims are substantially same as or equivalent to claim 77.

Consequently, claims 1-13, 21-27 and 77-79 are classified into Invention 1.

[Chemical formula 1]

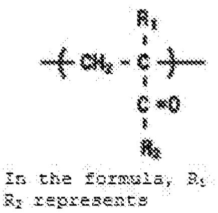

(1)

In the formula, $R_1$ represents H or $CH_3$.
$R_2$ represents

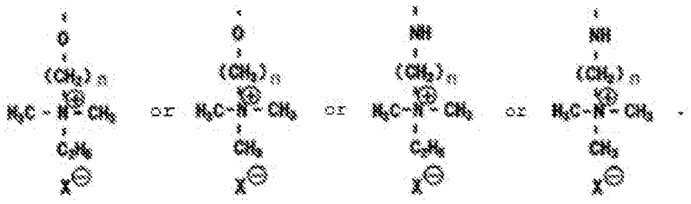

n is an integer of 1-10.
$X^-$ represents a halide ion, $C_2H_5OSO_3^-$, or $CH_3OSO_3^-$.

[Chemical formula 2]

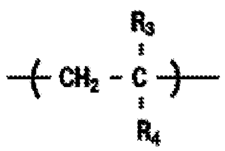

(2)

In the formula, R3 represents H or CH3.

(Continued to next extra sheet)

Form PCT/ISA/210 (extra sheet) (January 2015)

**INTERNATIONAL SEARCH REPORT**

International application No.
PCT/JP2015/084407

$R_4$ represents

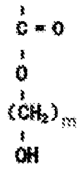 or 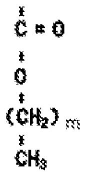 or 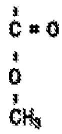 or 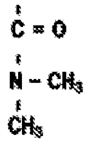 or 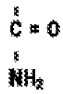 or

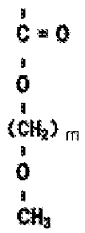 or 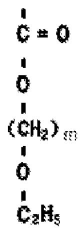 or 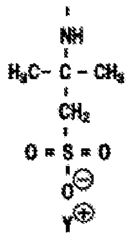 or 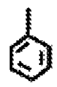 .

m is an integer of 1-10.
Y+ represents Na+ or K+.

(Invention 2) claims 14-20

Claims 14-20 are not dependent on claim 1.

Further, claims 14-20 have no relationship such that these claims are substantially same as or equivalent to any claim classified into Invention 1.

Consequently, claims 14-20 cannot be classified into Invention 1.

Claims 14-20 have a special technical feature wherein any of the members constituting the sanitary product includes 0.2-20 g/$m^2$ of a water-soluble cationic polymer that comprises a quaternary-ammonium-salt homopolymer or a quaternary-ammonium-salt copolymer and that has a streaming potential of 1,500 μeq/L or greater and a molecular weight of 2,000 or higher. Claims 14-20 are hence classified as Invention 2.

(Invention 3) claims 28-57

Claims 28-57 are not dependent on claim 1.

Further, claims 28-57 have no relationship such that these claims are substantially same as or equivalent to any claim classified into Invention 1 or Invention 2.

Consequently, claims 28-57 cannot be classified into either Invention 1 or Invention 2.

Claims 28-57 have a special technical feature wherein the water-soluble cationic polymer has been disposed nearer to the skin side than the constituent member, which is present nearer to the skin side than a highly absorbing polymer, and has an aggregation rate of 0.75 mPa·s/s or less. Claims 28-57 are hence classified as Invention 3.

(Continued to next extra sheet)

INTERNATIONAL SEARCH REPORT

International application No. PCT/JP2015/084407

(Invention 4) claims 58-76 and 80

Claims 58-76 and 80 are not dependent on claim 1.

Further, claims 58-76 and 80 have no relationship such that these claims are substantially same as or equivalent to any claim classified into Invention 1, Invention 2 or Invention 3.

Consequently, claims 58-76 and 80 cannot be classified into any one of Invention 1, Invention 2 and Invention 3.

Claims 58-76 and 80 have a special technical feature wherein the treating agent comprises a water-soluble cationic polymer having a molecular weight of 2,000 or higher, the cationic polymer having an inorganic/organic value, which is the ratio of the inorganic value to the organic value, of 0.6-4.6, the cationic polymer being a quaternary-ammonium-salt homopolymer, a quaternary-ammonium-salt copolymer, or a quaternary-ammonium-salt polycondensate. Claims 58-76 and 80 are hence classified as Invention 4.

Subject to be covered by this search:

Claim 6 states "the anionic polymerizable monomer is 2-acrylamido-2-methylpropanesulfonic acid, methacrylic acid, acrylic acid, or styrenesulfonic acid or is a salt of any of these compounds".

However, claim 4, on which claim 6 depends, includes no statement about "anionic polymerizable monomer".

Therefore, with respect to claim 6, a search was made on the assumption that the cationic polymerizable monomer is a product of copolymerization of a polymerizable monomer having a quaternary ammonium moiety with an anionic polymerizable monomer and that the anionic polymerizable monomer is 2-acrylamido-2-methylpropanesulfonic acid, methacrylic acid, acrylic acid, or styrenesulfonic acid or is a salt of any of these compounds.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002528232 A
- JP 2006511281 A
- JP 2009506056 A
- JP 2010540207 A
- JP 2003027371 A
- JP 4776407 B
- JP 4526271 B
- JP 4587928 B
- JP 4651392 B
- JP 4084278 B

### Non-patent literature cited in the description

- **ATSUSHI FUJITA.** Organic Analysis. Kaniya Shoten, 1930
- **ATSUSHI FUJITA.** Organic Qualitative Analysis: Systematic Pure Substances. Kyoritsu Shuppan Co., Ltd, 1953
- **ATSUSHI FUJITA.** Chemical Experiments - Organic Chemistry. Kawade Shobo, 1971
- **ATSUSHI FUJITA ; MASAMI AKATSUKA.** Systematic Organic Qualitative Analysis (Mixture). Kazamashobo, 1974
- **YOSHIO KOUDA ; SHIROH SATOH ; YOSHIO HOMMA.** Organic Conceptual Diagram, Basics and Applications. Sankyo Shuppan Co., Ltd, 2008